# EUROPÄISCHE PATENTSCHRIFT

(11) **EP 0 393 501 B1**
(45) Veröffentlichungstag und Bekanntmachung des Hinweises auf die Patenterteilung: **23.06.1993**
(21) Anmeldenummer: 90106991.4
(22) Anmeldetag: 11.04.1990
(51) Int. Cl.: C09K 19/30, C07C 69/618, C07D 319/06, C07D 239/26, C07D 213/89

(54) **Flüssigkristallkomponenten mit einer trans-3-Cyclohexylallyloxygruppe**
Liquid crystal components with a trans-3-cyclohexylallyloxy group
Constituants de cristal liquide avec un groupe trans-3-cyclohexylallyloxy

(30) Priorität: 20.04.1989 CH 1547/89
(43) Veröffentlichungstag der Anmeldung: 24.10.1990
(73) Patentinhaber: F. HOFFMANN-LA ROCHE AG, 4002 Basel (CH)
(72) Erfinder: Kelly, Stephen, Dr., CH-4314 Möhlin (CH)
(74) Vertreter: Cottong, Norbert A.

(56) Entgegenhaltungen:
- WO-A-87/04426
- US-A- 4 052 423
- PATENT ABSTRACTS OF JAPAN, Band 11, Nr. 115 (C-415)[2562], 10. April 1987, Seite 71 C 415; & JP-A-61 257 935

## Beschreibung

Die vorliegende Erfindung betrifft neue Verbindungen mit einer trans-3-Cyclohexylallyloxygruppe, flüssigkristalline Gemische, die solche Verbindungen enthalten sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), Gast/Wirt-Zellen, TN-Zellen ("twisted nematic") und STN-Zellen ("super-twisted nematic") mit verdrillt nematischer Struktur, SBE-Zellen ("super-birefringence effect"), Phase-Change-Zellen mit einem cholesterisch-nematischen Phasenübergang und OMI-Zellen ("optical mode interference"). Die gebräuchlichsten Anzeigevorrichtungen beruhen auf dem Schadt-Helfrich-Effekt und besitzen eine verdrillt nematische Struktur.

In Appl. Phys. Lett. 36, 899 (1980) und in Recent Developments in Condensed Matter Physics 4, 309 (1981) werden ferner elektro-optische Vorrichtungen auf der Basis von chiral getilteten smektischen Flüssigkristallen vorgeschlagen. Hierbei werden die ferroelektrischen Eigenschaften dieser Materialien ausgenutzt. Als getiltete smektische Phasen eignen sich beispielsweise smektisch C, F, G, H, I und K Phasen. Bevorzugt sind im allgemeinen smektisch C Phasen, welche besonders grosse Ansprechgeschwindigkeiten ermöglichen. Die chiral getilteten Phasen werden üblicherweise mit S_{C}*, S_{F}* etc. bezeichnet, wobei der Stern die Chiralität angibt.

Die Flüssigkristallmaterialien müssen eine gute chemische und thermische Stabilität und eine hohe Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben. Weiterhin sollen sie bei üblichen Betriebstemperaturen eine geeignete Mesophase besitzen, beispielsweise eine nematische, cholesterische oder chiral getiltete smektische Phase. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. Neben dem generellen Interesse an Flüssigkristallmaterialien mit hoher optischer Anisotropie besteht in letzter Zeit ein vermehrtes Interesse an Materialien mit niedriger optischer Anisotropie, insbesondere für aktiv adressierte Flüssigkristallanzeigen, z.B. bei TFT-Anwendungen (Dünnfilmtransistor) in Fernsehgeräten. Anderseits sollten chiral getiltete smektische Flüssigkristalle eine ausreichend hohe spontane Polarisation, aber eine vergleichsweise kleine Verdrillung besitzen. Die Ganghöhe (Pitch) der Verdrillung sollte vorzugsweise deutlich grösser sein als der Plattenabstand der verwendeten Zelle und typischerweise mindestens etwa 10 µm betragen, um gut schaltende, bistabile Displays zu erhalten.

Flüssigkristalle werden zwecks Optimierung der Eigenschaften in der Regel als Mischungen mehrerer Komponenten verwendet. Es ist daher wichtig, dass die Komponenten untereinander gut mischbar sind. Cholesterische Gemische können vorzugsweise aus einem oder mehreren optisch aktiven Dotierstoffen und einem nematischen Flüssigkristallmaterial bestehen, und ferroelektrische Flüssigkristalle können vorzugsweise aus einem oder mehreren optisch aktiven Dotierstoffen und einem Flüssigkristallmaterial mit getilteter smektischer Phase bestehen.

Gegenstand der vorliegenden Erfindung sind die Verbindungen der allgemeinen Formel 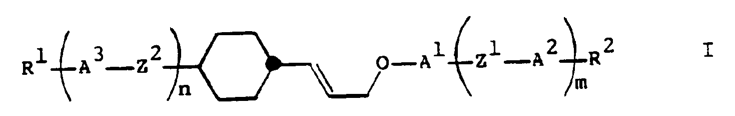
worin R¹ eine Gruppe R³ oder R³-A⁴-Z³- und R² ein Gruppe R⁴ oder -Z⁴-A⁵-R⁴ bezeichnen; m und n unabhängig voneinander für die Zahl O oder 1 stehen; A¹, A², A³, A⁴ und A⁵ unabhängig voneinander unsubstituiertes oder mit Halogen, Cyano und/oder Methyl substituiertes 1,4-Phenylen, in welchem gegebenenfalls 1 oder 2 CH-Gruppen durch Stickstoff ersetzt sind, trans-1,4-Cyclohexylen, in welchem gegebenenfalls 2 nicht benachbarte Methylengruppen durch Sauerstoff und/oder Schwefel ersetzt sind, 1-Cyano-trans-1,4-cyclohexylen, Bicyclo[2.2.2]octan-1,4-diyl, Naphthalin-2,6-diyl, Tetralin-2,6-diyl oder trans-Decalin-2,6-diyl darstellen; Z¹, Z², Z³ und Z⁴ unabhängig voneinander eine einfache Kovalenzbindung, -COO-, -OOC-, -CH₂O-, -OCH₂-, -CH₂CH₂-, -C≡C-, -(CH₂)₃O-, -O(CH₂)₃- oder die trans-Form von -CH=CH-CH₂O- oder -OCH₂-CH=CH- bedeuten; R³ und R⁴ unabhängig voneinander Halogen, Cyano, -NCS, -CF₃, -OCF₃ oder Alkyl bezeichnen, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist; und X Halogen, Cyano oder Methyl bedeutet.

Die Verbindungen der Formel I besitzen trotz der vergleichsweise hohen Flexibilität der trans-3-Cyclohexylallyloxygruppe eine ausgeprägte Tendenz zur Ausbildung von flüssigkristallinen Phasen. Die optisch inaktiven Verbindungen der Formel I besitzen meist eine nematische, eine smektische A und/oder eine getiltete smektische (vor allem S_{C}) Phase, und die optisch aktiven Verbindungen der Formel I besitzen meist eine cholesterische, eine smektisch A und/oder eine chiral getiltete smektische (vor allem S_{C}*) Phase. Diese Mesophasentypen sind besonders geeignet zur Erzielung von nematischen, cholesterischen oder chiral getilteten smektischen Phasen in Flüssigkristallgemischen. Die vorliegende Erfindung bietet somit eine breite Palette neuer Komponenten und Mischungen für die üblichen elektrooptischen Anwendungsbereiche von Flüssigkristallen.

Die Verbindungen der Formel I besitzen eine hohe chemische Stabilität und eine hohe Stabilität gegenüber elektrischen und magnetischen Feldern. Sie sind farblos, leicht herstellbar und sehr gut löslich untereinander und in bekannten Flüssigkristallmaterialien. Ferner besitzen sie tiefe Viskositäten und ergeben in Anzeigevorrichtungen kurze Ansprechzeiten.

Die Eigenschaften der Verbindungen der Formel I können je nach Zahl und Bedeutung der Ringe und der Substituenten in breiten Bereichen variiert werden. Beispielsweise führen aromatische Ringe zu höheren und gesättigte Ringe zu tieferen Werten der optischen Anisotropie. Eine Erhöhung des Klärpunktes kann beispielsweise durch Einführung eines oder mehrerer weiterer Ringe erreicht werden. Polare Endgruppen, wie Cyano, Halogen, -NCS, -CF₃ oder -OCF₃, und Ringe wie Pyrimidin-2,5-diyl, trans-1,3-Dioxan-2,5-diyl etc. erhöhen die dielektrische Anisotropie, Ringe wie Pyridazin-3,6-diyl, 1-Cyano-trans-1,4-cyclohexylen, 2,3-Dicyano-1,4-phenylen etc. vermindern die dielektrische Anisotropie und laterale Halogen- und Cyanosubstituenten ergeben eine Beitrag zur Dielektrizitätskonstante sowohl parallel als auch senkrecht zur Moleküllängsachse, was je nach Substitutionsmuster zur Erhöhung oder Verminderung der dielektrischen Anisotropie ausgenützt werden kann. Ferner können durch laterale Substituenten an einem oder mehreren Ringen die Mesophasebereiche modifiziert, eine allfällige Tendenz zur Ausbildung hochgeordneter smektischer Phasen weitgehend unterdrückt und oft auch die Löslichkeit verbessert werden. Weiterhin können durch eine C=C-Doppelbindung in der Seitenkette die elastischen Eigenschaften, die Schwellenspannungen, die Ansprechzeiten, die Mesophasen etc. weiter modifiziert werden.

Die erfindungsgemässen Verbindungen ermöglichen daher eine weitere Optimierung der Flüssigkristallgemische und eine Modifizierung der elektro-optischen Eigenschaften in einem weiten Bereich je nach gewünschten Eigenschaften.

Der Ausdruck "Halogen" bezeichnet im Rahmen der vorliegenden Erfindung Fluor, Chlor, Brom oder Jod, insbesondere Fluor oder Chlor.

Der Ausdruck "unsubstituiertes oder mit Halogen, Cyano und/oder Methyl substituiertes 1,4-Phenylen, in welchem gegebenenfalls 1 oder 2 CH-Gruppen durch Stickstoff ersetzt sind," umfasst Gruppen wie 1,4-Phenylen, 2-Fluor-1,4-phenylen, 2,3-Difluor-1,4-phenylen, 2-Chlor-1,4-phenylen, 2-Brom-1,4-phenylen, 2-Cyano-1,4-phenylen, 2,3-Dicyano-1,4-phenylen, 2-Methyl-1,4-phenylen, Pyridin-2,5-diyl, Pyrazin-2,5-diyl, Pyrimidin-2,5-diyl, Pyridazin-3,6-diyl und dergleichen. Bevorzugte Gruppen sind 1,4-Phenylen, 2-Fluor-1,4-phenylen, 2,3-Difluor-1,4-phenylen, Pyridin-2,5-diyl, Pyrazin-2,5-diyl und Pyrimidin-2,5-diyl.

Der Ausdruck "trans-1,4-Cyclohexylen, in welchem gegebenenfalls 2 nicht benachbarte Methylengruppen durch Sauerstoff und/oder Schwefel ersetzt sind," umfasst Gruppen wie trans-1,4-Cyclohexylen, trans-1,3-Dioxan-2,5-diyl und trans-1,3-Dithian-2,5-diyl.

Der Ausdruck "Tetralin-2,6-diyl" bezeichnet 1,2,3,4-Tetrahydronaphthalin-2,6-diyl. Der Ausdruck "Decalin-2,6-diyl" umfasst von Decahydronaphthalin abgeleitete, 2,6-disubstituierte Gruppen, insbesondere (4aαH,8aβH)-Decahydronaphthalin-2α,6β-diyl.

Der Ausdruck "Alkyl, in welchem gegebenenfalls eine >CH-CH< durch >C=C< ersetzt ist und/oder gegebenenfalls eine und/oder zwei nicht benachbarte Methylengruppen durch -O-, -COO- und/oder -OOC- ersetzt sind und/oder gegebenenfalls eine Methylengruppe durch -CHX- ersetzt ist," umfasst geradkettige und verzweigte (gegebenenfalls chirale) Reste wie Alkyl, 1E-Alkenyl, 3E-Alkenyl, 4-Alkenyl, Alkenyl mit endständiger Doppelbindung, Alkoxy, 2E-Alkenyloxy, 3-Alkenyloxy, Alkenyloxy mit endständiger Doppelbindung, Alkoxyalkyl, Alkenyloxyalkyl, Alkoxycarbonyl, Alkoxycarbonylalkoxy, Alkanoyloxy, 1-Haloalkyl, 2-Haloalkyl, 2-Haloalkoxy, 2-Haloalkoxycarbonyl, 1-Cyanoalkyl, 2-Cyanoalkyl, 2-Cyanoalkoxy, 2-Cyanoalkoxycarbonyl, 1-Methylalkyl, 2-Methylalkyl, 1-Methylalkoxy, 2-Methylalkoxy, 2-Methylalkoxycarbonyl und dergleichen. Beispiele bevorzugter Reste sind Methyl, Aethyl, Propyl, Butyl, Pentyl, Hexyl, Heptyl, Octyl, Nonyl, Decyl, Undecyl, Dodecyl, 1-Methylpropyl, 1-Methylheptyl, 2-Methylbutyl, 3-Methylpentyl, Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4Z-Heptenyl, 5-Hexenyl, 6-Heptenyl, 7-Octenyl, 8-Nonenyl, 9-Decenyl, 10-Undecenyl, 11-Dodecenyl, Methoxy, Aethoxy, Propyloxy, Butyloxy, Pentyloxy, Hexyloxy, Heptyloxy, Octyloxy, Nonyloxy, Decyloxy, Undecyloxy, Dodecyloxy, 1-Methylpropyloxy, 1-Methylheptyloxy, 2-Methylbutyloxy, 3-Methylpentyloxy, Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy, 7-Octenyloxy, 8-Nonenyloxy, 9-Decenyloxy, 10-Undecenyloxy, 11-Dodecenyloxy, Methoxymethyl, Aethoxymethyl, Propyloxymethyl, Allyloxymethyl, Methoxycarbonyl, Aethoxycarbonyl, Propyloxycarbonyl, 1-Methylpropyloxycarbonyl, 1-(Methoxycarbonyl)äthoxy, 1-(Aethoxycarbonyl)äthoxy, Acetoxy, Propionyloxy, Butyryloxy, 1-Fluorpropyl, 1-Fluorpentyl, 1-Chlorpropyl, 2-Fluorpropyl, 2-Fluorpentyl, 2-Chlorpropyl, 2-Fluorpropyloxy, 2-Fluorbutyloxy, 2-Fluorpentyloxy, 2-Fluorhexyloxy, 2-Chlorpropyloxy, 2-Fluorbutyloxy, 2-Fluorpropyloxycarbonyl, 2-Fluorbutyloxycarbonyl, 2-Fluorpentyloxycarbonyl, 2-Fluor-3-methylbutyloxycarbonyl, 2-Fluor-4-methylpentyloxycarbonyl, 2-Chlorpropyloxycarbonyl, 1-Cyanopropyl, 1-Cyanopentyl, 2-Cyanopropyl, 2-Cyanopentyl, 2-Cyanopropyloxy, 2-Cyanobutyloxy, 2-Cyanopentyloxy, 2-Cyanohexyloxy, 2-Cyanopropyloxycarbonyl, 2-Cyanobutyloxycarbonyl, 2-Cyano-3-methylbutyloxycarbonyl, 2-Cyano-4-methylpentyloxycarbonyl und dergleichen.

Im allgemeinen sind diejenigen Verbindungen der Formel I bevorzugt, worin die Ringe A¹, A², A³, A⁴ und A⁵ unabhängig voneinander für unsubstituiertes oder mit Halogen, Cyano und/oder Methyl substituiertes 1,4-Phenylen oder für trans-1,4-Cyclohexylen stehen oder einer der Ringe A¹ - A⁵ (vorzugsweise Ring A²) auch einen der oben genannten Heterocyclen bedeutet und/oder einer der Ringe A¹ - A⁵ (vorzugsweise Ring A²) auch einen Ring ausgewählt aus der Gruppe bestehend aus 1-Cyano-trans-1,4-cyclohexylen, Bicyclo[2.2.2]octan-1,4-diyl, Naphthalin-2,6-diyl, Tetralin-2,6-diyl und trans-Decalin-2,6-diyl bedeutet.

Ein bevorzugter Aspekt betrifft die Verbindungen der Formel I, worin Ring A³ trans-1,4-Cyclohexylen darstellt, Z² eine einfache Kovalenzbindung oder -CH₂CH₂- bedeutet und n für die Zahl O oder 1 steht.

Ring A¹ in obiger Formel I steht vorzugsweise für unsubstituiertes oder mit Halogen, Cyano und/oder Methyl substituiertes 1,4-Phenylen oder, wenn eine niedrige optische Anisotropie gewünscht ist, auch für trans-1,4-Cyclohexylen.

Ein bevorzugter Aspekt betrifft somit die Verbindungen der allgemeinen Formel 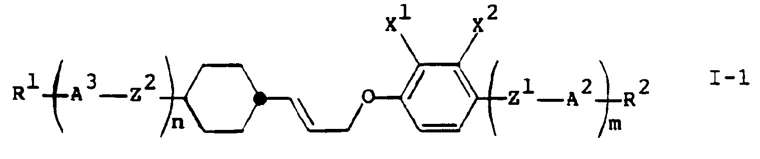
worin X¹ und X² unabhängig voneinander Wasserstoff, Halogen, Cyano oder Methyl bezeichnen und A², A³, R¹, R², Z¹, Z², m und n die obigen Bedeutungen haben,
insbesondere die Verbindungen der allgemeinen Formel 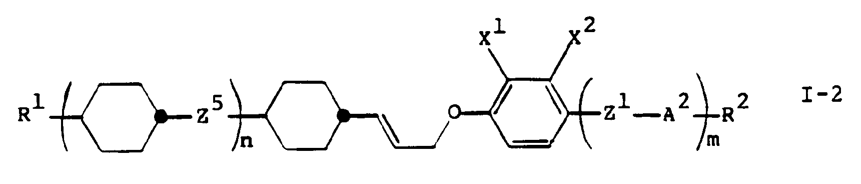
worin Z⁵ eine einfache Kovalenzbindung oder -CH₂CH₂-bezeichnet, X¹ und X² unabhängig voneinander Wasserstoff, Halogen, Cyano oder Methyl darstellen, und R¹, R², Z¹, m, n und A² die obigen Bedeutungen haben.

Ein weiterer bevorzugter Aspekt betrifft die Verbindungen der allgemeinen Formel 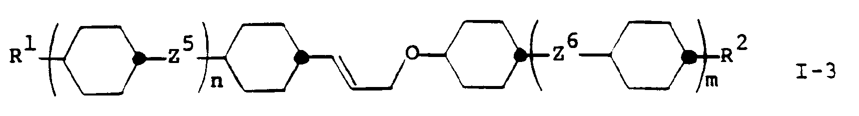
worin Z⁵ und Z⁶ unabhängig voneinander eine einfache Kovalenzbindung oder -CH₂CH₂- bezeichnen, und R¹, R², m und n die obigen Bedeutungen haben.

Im allgemeinen sind diejenigen Verbindungen der Formeln I, I-1, I-2 und I-3 bevorzugt, worin R¹ eine Gruppe R³ und/oder R² eine Gruppe R⁴ bezeichnen. Besonders bevorzugt sind im allgemeinen die bicyclischen Verbindungen (R¹=R³, R²=R⁴, m=n=O) und die tricyclischen Verbindungen (R¹=R³, R²=R⁴ und m=1, n=O bzw. m=O, n=1).

Die Verbindungen mit mindestens 4 Ringen - d.h. die Verbindungen der obigen Formeln I, I-1, I-2 und I-3, worin m und n die Zahl 1 bezeichnen und/oder R¹ eine Gruppe R³-A⁴-Z³- bezeichnet und/oder R² eine Gruppe -Z⁴-A⁵-R⁴ bezeichnet, - besitzen jedoch deutlich höhere Klärpunkte und tiefere Dampfdrücke. Sie eignen sich daher insbesondere als Dotierstoffe zur Klärpunktserhöhung oder als stationäre Phasen in der Gaschromatographie. Bevorzugt sind hierbei diejenigen Verbindungen, worin A⁴ und A⁵ unabhängig voneinander 1,4-Phenylen oder trans-1,4-Cyclohexylen bezeichnen und Z³ und Z⁴ je eine einfache Kovalenzbindung bezeichnen, d.h. diejenigen Verbindungen der obigen Formeln I, I-1, I-2 und I-3, worin R¹ eine Gruppe R³, 4-R³-Phenyl oder trans-4 -R³-Cyclohexyl bezeichnet und R² eine Gruppe R⁴, 4-R⁴-Phenyl oder trans-4-R⁴-Cyclohexyl bezeichnet.

Z¹ - Z⁴ stehen in den obigen Formel I, I-1, I-2 und I-3 vorzugsweise je für eine einfache Kovalenzbindung oder eine der Gruppen Z¹, Z², Z³ und Z⁴ auch für -COO-, -OOC-, -CH₂O-, -OCH₂-, -CH₂CH₂-, -C≡C-, -(CH₂)₃O-, -O(CH₂)₃- oder die trans-Form von -CH=CH-CH₂O- oder -OCH₂-CH=CH-.

Ferner sind im allgemeinen diejenigen Verbindungen der Formeln I, I-1, I-2 und I-3 bevorzugt, welche höchstens 1 oder 2 laterale Substituenten aufweisen. Bevorzugte laterale Substituenten sind Cyano und insbesondere Fluor.

Beispiele besonders bevorzugter Untergruppen von Verbindungen der Formel I sind die Verbindungen der allgemeinen Formeln 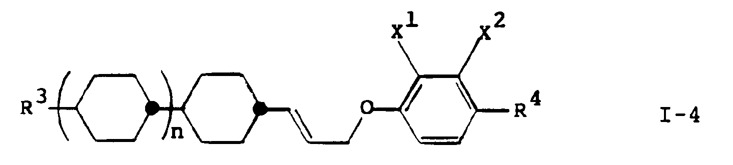 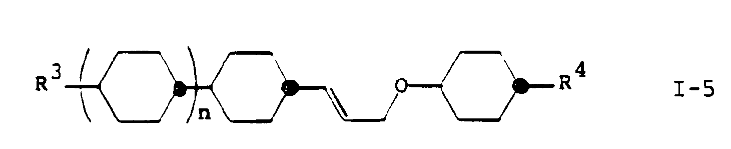 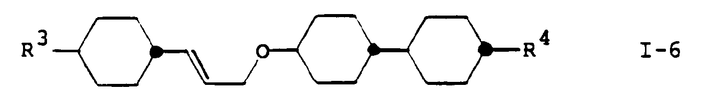 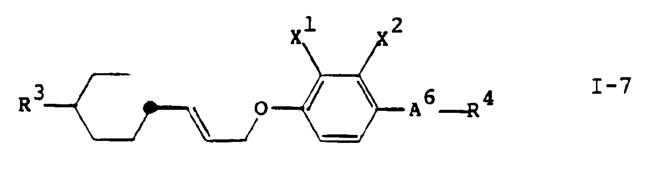 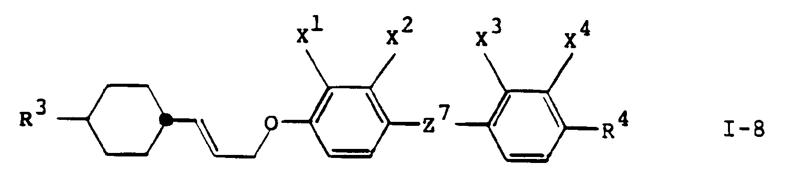 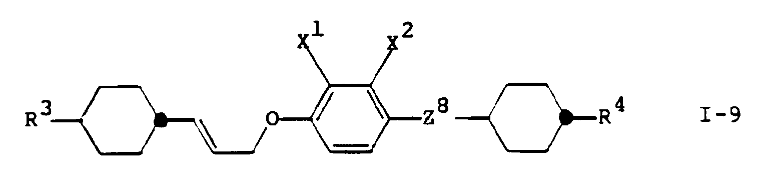
worin n für die Zahl O oder 1 steht; X¹, X², X³ und X⁴ unabhängig voneinander Wasserstoff, Halogen, Cyano oder Methyl bedeuten; A⁶ 1,4-Phenylen, Pyridin-2,5-diyl, Pyrimidin-2,5-diyl, Pyrazin-2,5-diyl, trans-1,4-Cyclohexylen, trans-1,3-Dioxan-2,5-diyl oder trans-Decalin-2,6-diyl darstellt; Z⁷ eine einfache Kovalenzbindung, -COO- oder -OOC- bedeutet; Z⁸ eine einfache Kovalenzbindung, -OOC-, -OCH₂-, -C≡C-, -O(CH₂)₃- oder die trans-Form von -OCH₂-CH=CH- bezeichnet; und R³ und R⁴ die obigen Bedeutungen haben.

In den obigen Formeln I-1, I-2, I-4, I-7, I-8 und I-9 stehen vorzugsweise X¹, X², X³ und X⁴ unabhängig voneinander für Wasserstoff, Fluor oder Cyano, insbesondere für Wasserstoff oder Fluor.

R³ und R⁴ in den obigen Formeln I und I-1 bis I-9 besitzen vorzugsweise höchstens je etwa 18 (insbesondere höchstens je etwa 12) Kohlenstoffatome. Ferner bedeutet vorzugsweise höchstens einer der Reste R³ und R⁴ Halogen, Cyano, -NCS, -CF₃ oder -OCF₃. R³ und R⁴ bedeuten somit unabhängig voneinander vorzugsweise eine C₁-C₁₈-Alkyl- oder C₂-C₁₈-Alkenylgruppe, in welcher gegebenenfalls eine oder zwei nicht benachbarte Methylengruppen durch -O-, -COO- und/oder -OOC- ersetzt sind, und/oder gegebenenfalls eine Methylengruppe durch -CHX- ersetzt ist, oder einer der Reste R³ und R⁴ kann auch Halogen, Cyano, -NCS, -CF₃ oder -OCF₃ bedeuten.

Für nematische und cholesterische Anwendungen sind im allgemeinen kurze Reste R³ und R⁴ (z.B. Reste mit höchstens 12, vorzugsweise höchstens 7 Kohlenstoffatomen) bevorzugt und vorzugsweise kann auch einer der Reste Halogen, Cyano, -NCS, -CF₃ oder -OCF₃ bedeuten. Für smektische Anwendungen (insbesondere getiltete smektische Phasen) sind im allgemeinen diejenigen Verbindungen bevorzugt, worin R³ und R⁴ unabhängig voneinander Alkyl bezeichnen, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist, und die Summe der Kohlenstoffatome in R³ und R⁴ zusammen mindestens 10, vorzugsweise mindestens 12 beträgt.

Bevorzugte Reste R³ sind Alkyl, Alkenyl, Alkoxy, Alkenyloxy, Alkoxycarbonyl, Alkenyloxycarbonyl, Alkanoyloxy und Alkenoyloxy, insbesondere Alkyl und Alkenyl. Besonders bevorzugt sind im allgemeinen Reste R³ mit bis zu 12 Kohlenstoffatomen. Bevorzugte Reste R⁴ sind Alkyl, Alkenyl, Alkoxy, Alkenoyloxy, Alkoxycarbonyl, Alkenyloxycarbonyl, Alkanoyloxy und Alkenoyloxy (insbesondere Alkyl, Alkenyl, Alkoxy und Alkenyloxy) sowie Halogen (insbesondere Fluor und Chlor), Cyano, -NCS, -CF₃ und -OCF₃. Besonders bevorzugt sind im allgemeinen Reste R⁴ mit bis zu 12 Kohlenstoffatomen.

Geradkettige Reste R³ bzw. R⁴ sind im allgemeinen bevorzugt. Um beispielsweise chirale Dotierstoffe für cholesterische oder für chiral getiltete smektische Flüssigkristalle zu erhalten, können jedoch vorzugsweise auch einer oder beide Reste R³ und R⁴ verzweigt-kettig chiral sein und/oder eine Gruppe -CHX-, worin X Halogen (vorzugsweise Fluor oder Chlor), Cyano oder Methyl bedeutet, anstelle einer Methylengruppe aufweisen. Um für chiral getiltete smektische Anwendungen eine hohe spontane Polarisation zu erhalten, sollte das Chiralitätszentrum (d.h. die Kettenverzweigung oder der Halogen- oder Cyanosubstituent) vorzugsweise nahe am Ringsystem, beispielsweise in 1- oder 2-Stellung des Restes R³ bzw. R⁴ sein. Ferner bleibt die Tendenz zur Bildung flüssigkristalliner Phasen grundsätzlich erhalten, wenn 1 oder 2 nicht benachbarte Methylengruppen durch -O-, -COO- und/oder -OOC- ersetzt werden.

Die Mesophasenbereiche, die Schwellenspannung, die Ansprechgeschwindigkeit, die Steilheit der Transmissionskennlinie etc. können ferner variiert werden durch Wahl der Stellung der C=C-Doppelbindung in ungesättigten Resten wie Alkenyl, Alkenyloxy und dergleichen. Der Effekt ist grundsätzlich bekannt z.B. aus Mol. Cryst. Liq. Cryst. 122, 241 (1985), 131, 109 (1985) und 148, 123 (1987). Bevorzugt sind Reste, welche die Doppelbindung in 1-Stellung (insbesondere E-Isomer), in 3-Stellung (insbesondere E-Isomer) oder in 4-Stellung (insbesondere Z-Isomer) der Kette unter Einschluss allfälliger Heteroatome aufweisen, wie 1E-Alkenyl, 3E-Alkenyl, 4Z-Alkenyl, 2E-Alkenyloxy, 3Z-Alkenyloxy und dergleichen. Ferner kann die Doppelbindung vorzugsweise auch in endständiger Position stehen, insbesondere im Falle von Verbindungen für smektische Anwendungen. Beispiele bevorzugter Reste mit endständiger Stellung der Doppelbindung sind 6-Heptenyl, 7-Octenyl, 8-Nonenyl, 9-Decenyl, 10-Undecenyl, 11-Dodecenyl, 5-Hexenyloxy, 6-Heptenyloxy, 7-Octenyloxy, 8-Nonenyloxy, 9-Decenyloxy, 10-Undecenyloxy, 11-Dodecenyloxy und dergleichen.

Die Verbindungen der Formel I können in an sich bekannter Weise hergestellt werden, beispielsweise durch Verätherung eines Allylalkohols der allgemeinen Formel 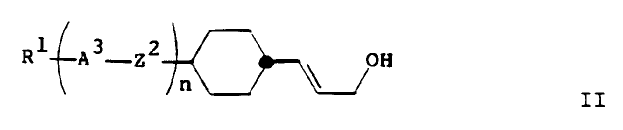 mit einer Verbindung der allgemeinen Formel

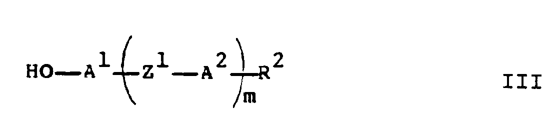

worin A¹, A², A³, R¹, R², Z¹, Z², m und n die obigen Bedeutungen haben.

Die Verätherung kann vorzugsweise in Gegenwart von Azodicarbonsäure-dialkylester (z.B. Azodicarbonsäure-diäthylester) und Triphenylphosphin in einem inerten organischen Lösungsmittel, beispielsweise einem Aether wie Tetrahydrofuran, Dioxan, Diäthyläther und dergleichen, erfolgen. Gewünschtenfalls kann die Verbindung der Formel II auch nach üblichen Methoden zuerst in ein Halogenid (vorzugsweise das Bromid) umgesetzt und dann veräthert werden. Letztere Methode ist bevorzugt, wenn A¹ einen gesättigten Ring (insbesondere trans-1,4-Cyclohexylen) darstellt.

Enthält eine Verbindung der Formel I eine oder mehrere Estergruppen in R¹, R², Z¹ oder Z², so kann die Veresterung vorzugsweise nach der Verätherung zum Allyläther erfolgen. Allfällige Aethergruppen in R¹, R², Z¹ oder Z² [d.h. -O- in R³ und R⁴ und Brückengruppen -CH₂O-, -OCH₂-, -(CH₂)₃O-, -O(CH₂)₃-, -CH=CH-CH₂O- und -OCH₂-CH=CH-] oder Dioxan- und Dithianringe können vor oder nach der obigen Verätherung zum Allyläther gebildet werden.

Die Verbindungen der Formel III, welche eine Brückengruppe -CH=CH-CH₂O- oder -OCH₂-CH=CH- aufweisen, können in analoger Weise zu den unten für die Verbindungen der Formeln I und II beschriebenen Methoden hergestellt werden. Die übrigen Verbindungen der Formel III sind bekannte oder Analoge bekannter Verbindungen und können nach bekannten Methoden hergestellt werden.

Die Verbindungen der Formel II sind neu. Die Herstellung der Verbindungen der Formeln I und II wird anhand repräsentativer Beispiele in den Schemata 1 - 3 illustriert, worin A², A³, R¹, R², R³, R⁴, X¹, X², X³, X⁴, Z¹, Z², m und n die obigen Bedeutungen haben und R⁵ Alkyl (bevorzugt C₁-C₁₀-Alkyl, besonders bevorzugt C₁-C₅-Alkyl, wie Methyl, Aethyl, Propyl, Butyl oder Pentyl) bezeichnet:

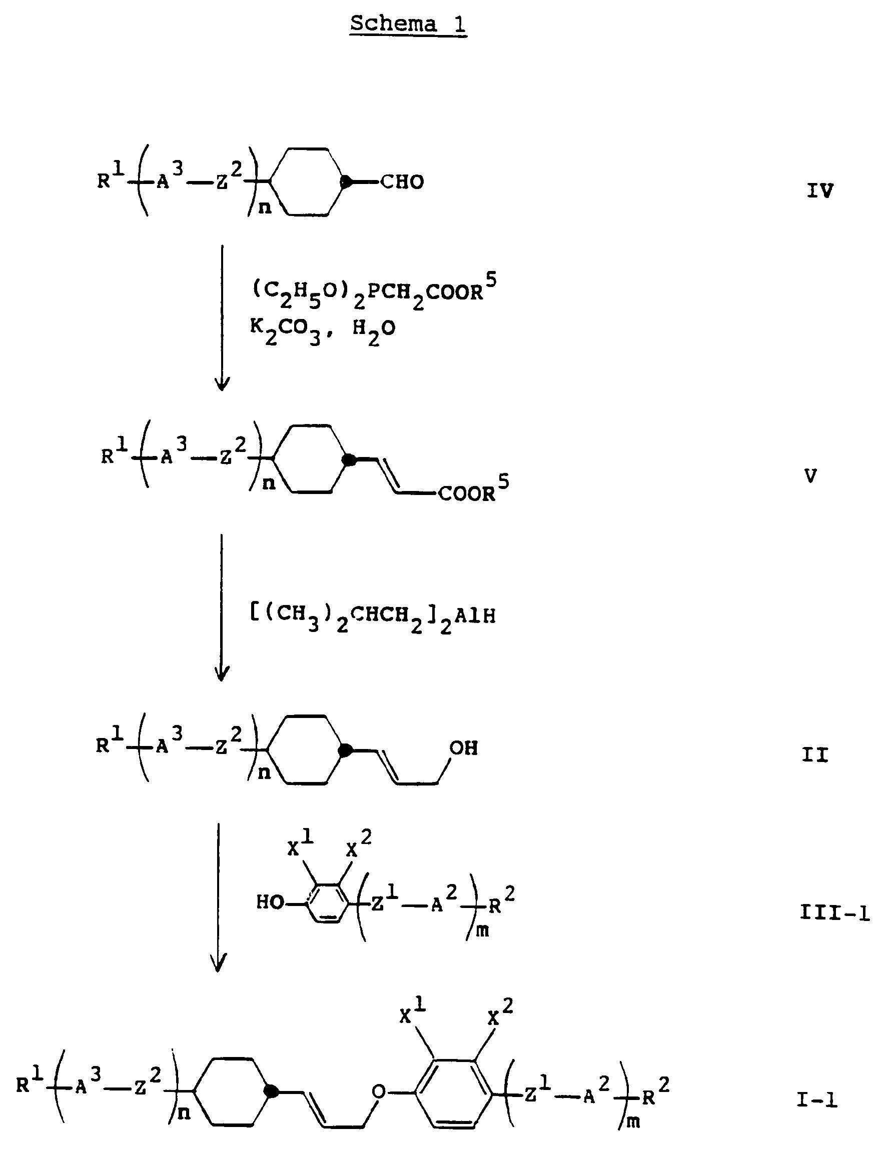

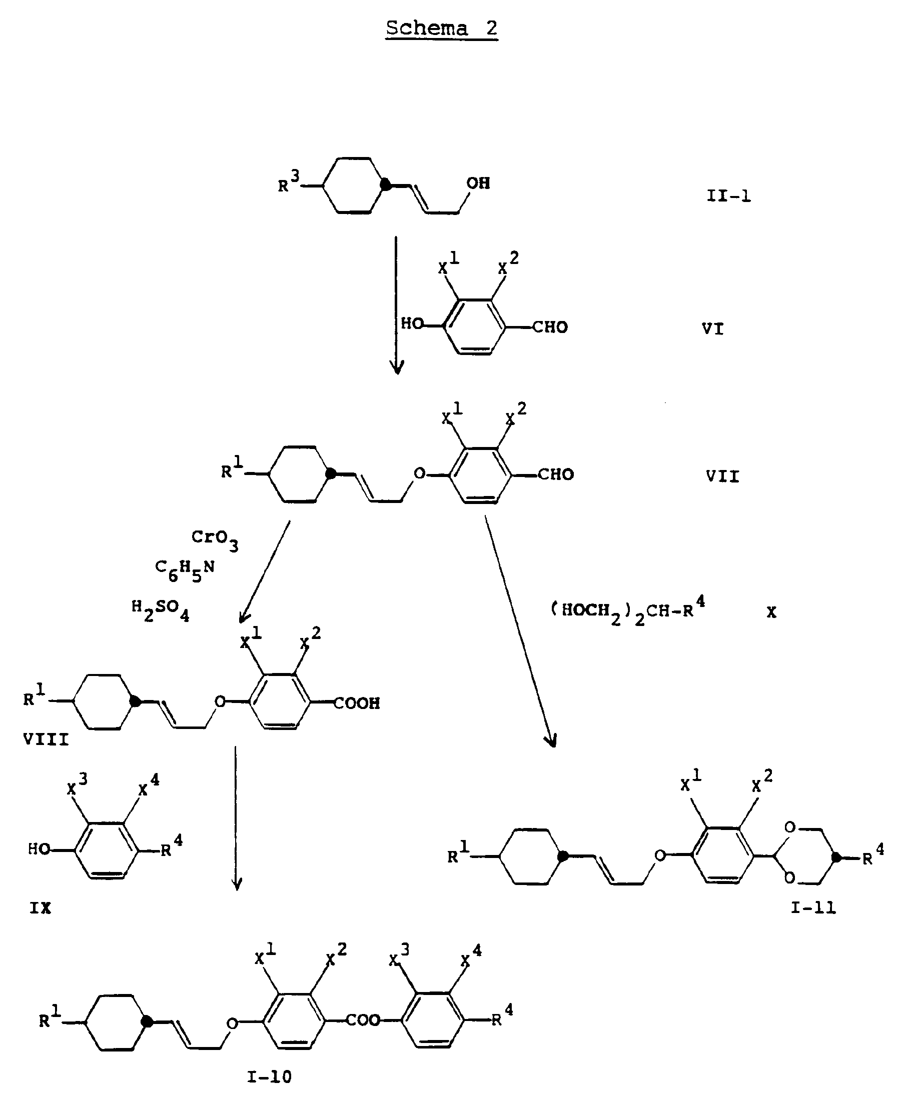

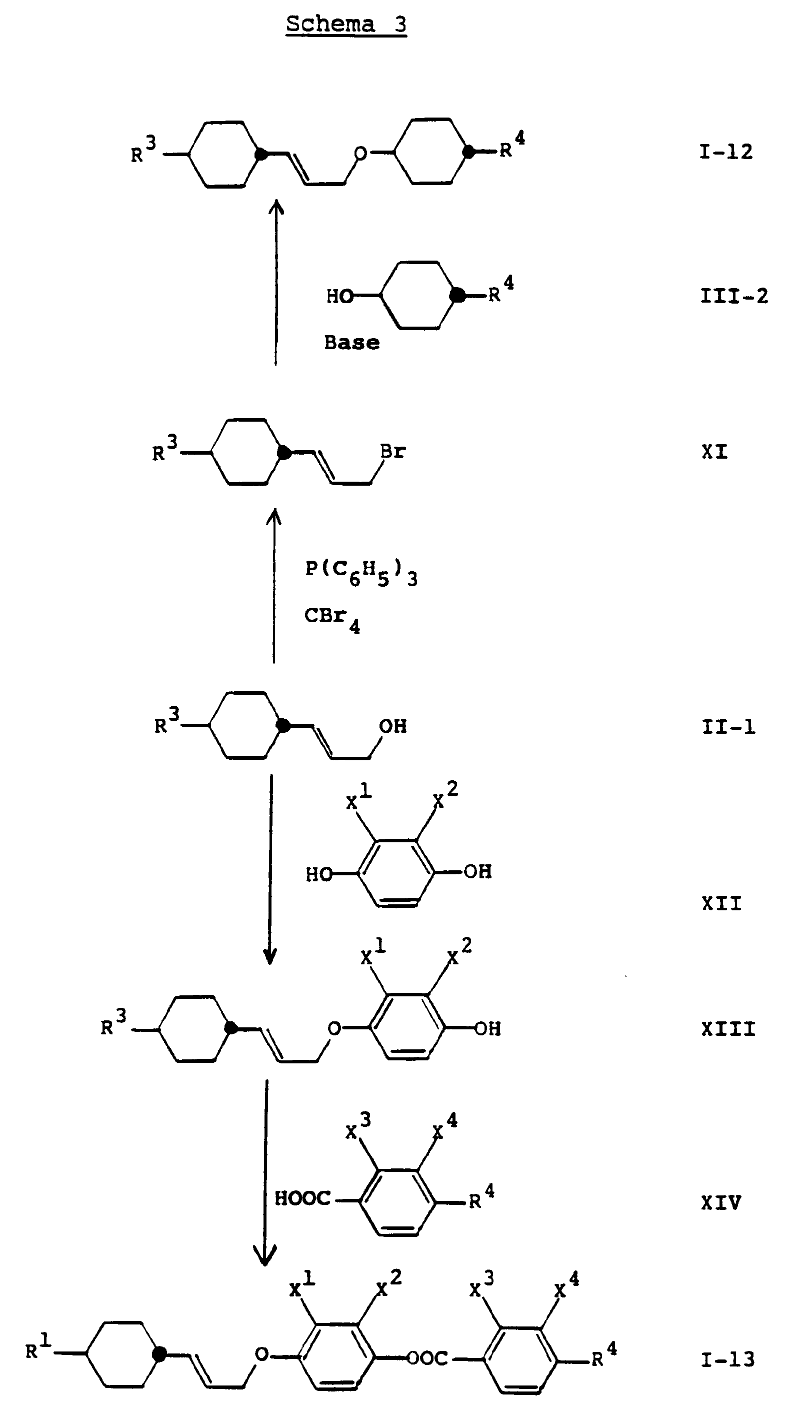

Die in den Schemata verwendeten Ausgangsmaterialien sind bekannte oder Analoge bekannter Verbindungen und können nach bekannten Methoden hergestellt werden. Solche Verbindungen wurden insbesondere schon verschiedentlich als Zwischenprodukte zur Herstellung anderer Flüssigkristallkomponenten beschrieben.

Die obigen Synthesemethoden sind besonders geeignet für Verbindungen, welche in R¹, R², R³, R⁴, Z¹ und Z² keine Estergruppe aufweisen. Verbindungen mit Estergruppen können hingegen vorzugsweise auch dadurch erhalten werden, dass man sinngemäss entsprechende Verbindungen verwendet, welche jeweils anstelle der Estergruppe eine Hydroxy- bzw. Carboxygruppe oder eine in Hydroxy bzw. Carboxy überführbare Gruppe aufweisen, und die Ueberführung in den Ester erst nach der Verätherung zum Allyläther durchführt. Entsprechende Methoden sind in den Schemata 2 und 3 illustriert.

Ferner kann zur Herstellung von Verbindungen mit einem Dioxan- oder Dithianring von einem Ausgangsmaterial mit einem Dioxan- oder Dithianring ausgegangen werden oder es kann auch (wie in Schema 2 illustriert) der Dioxan- oder Dithianring erst nach der Verätherung zum Allyläther gebildet werden.

Die Umsetzung der Verbindung der Formel II zur Verbindung der Formel I-1 und die Umsetzung der Verbindung der Formel II-1 zur Verbindung der Formel VII bzw. XIII kann vorzugsweise in Gegenwart von Azodicarbonsäure-dialkylester und Triphenylphosphin erfolgen. Gewünschtenfalls kann eine Hydroxygruppe der Verbindung der Formel XII geschützt werden durch Verätherung, z.B. zur Tetrahydropyranyloxygruppe.

Die Veresterung der Verbindung der Formel VIII zur Verbindung der Formel I-10 bzw. der Verbindung der Formel XIII zur Verbindung der Formel I-13 kann vorzugsweise in Gegenwart von N,N'-Dicyclohexylcarbodiimid und 4-(Dimethylamino)pyridin erfolgen.

Die Verätherung der Verbindung der Formel XI kann in Gegenwart üblicher Basen, wie Lithiumhydrid, Natriumhydrid und dergleichen, erfolgen.

Die Verbindungen der Formel V, worin n für die Zahl O und R¹ für eine Gruppe R³ stehen, sind bekannt aus EP-A-O1O7116 oder Analoge dieser Verbindungen.

Die bicyclischen und tricyclischen Verbindungen der Formel V, worin n für die Zahl O oder 1 steht und R¹ eine Gruppe R³-A⁴-Z³- bezeichnet oder n für die Zahl 1 steht und R¹ eine Gruppe R³ bezeichnet, sind hingegen neu und ebenfalls Gegenstand der vorliegenden Erfindung. Sie sind ebenfalls geeignete Flüssigkristallkomponenten und besitzen grösstenteils selbst flüssigkristalline Eigenschaften.

Verbindungen der Formel V können vorzugsweise direkt aus den entsprechenden Verbindungen der Formel IV erhalten werden. Dies gilt auch für Verbindungen der Formel V, welche eine oder mehrere Estergruppen in R³, Z² und/oder Z³ enthalten, wenn keine weitere Umsetzung zu Verbindungen der Formel I gewünscht ist. In analoger Weise zu oben Gesagtem kann in diesen Fällen aber auch von einer Verbindung mit einer Hydroxy- bzw. Carboxygruppe oder mit einer in Hydroxy bzw. Carboxy überführbaren Gruppe ausgegangen werden und nach Umsetzung zum Acrylsäureester die gewünschte Estergruppe eingeführt werden.

Besonders bevorzugte bicyclische oder tricyclische Verbindungen der Formel V sind die Verbindungen der allgemeinen Formel 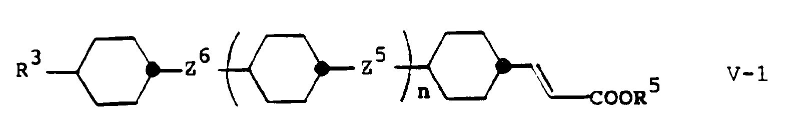
worin n für die Zahl O oder 1 steht, Z⁵ und Z⁶ unabhängig voneinander eine einfache Kovalenzbindung oder -CH₂CH₂- darstellen, R⁵ Alkyl bezeichnet und R³ die obige Bedeutung hat.

Die Verbindungen der Formel V-1 besitzen eine sehr tiefe optische Anisotropie und zugleich erstaunlich breite nematische Phasen (bzw. im Falle optisch aktiver Verbindungen cholesterische Phasen) mit hohen Klärpunkten.

Bevorzugte Reste R³ in den Formeln V und V-1 sind Alkyl, Alkenyl, Alkoxy, Alkenyloxy, Alkoxymethyl, Alkenyloxymethyl und 2-(Alkoxycarbonyl)vinyl, insbesondere Alkyl. Vorzugsweise besitzen diese Reste bis zu 12, insbesondere bis zu 7 Kohlenstoffatome. Geradkettige Reste R³ sind im allgemeinen bevorzugt.

R⁵ in den obigen Formeln V und V-1 steht vorzugsweise für C₁-C₁₀-Alkyl, insbesondere für C₁-C₅-Alkyl (z.B. Aethyl). Geradkettige Reste R⁵ sind im allgemeinen bevorzugt. Ferner steht vorzugsweise eine oder beide der Gruppen Z⁵ und Z⁶ in Formel V-1 für eine einfache Kovalenzbindung.

Die Verbindungen der Formeln I und V können in Form von Gemischen untereinander und/oder mit andern Flüssigkristallkomponenten verwendet werden. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, und viele davon sind zudem im Handel erhältlich.

Die Erfindung betrifft daher ebenfalls ein flüssigkristallines Gemisch mit mindestens 2 Komponenten, welches dadurch gekennzeichnet ist, dass mindestens eine Komponente eine Verbindung der Formel I (insbesondere eine der als bevorzugt genannten Verbindungen) ist.

Aufgrund der guten Löslichkeit sowie anderseits der grossen Variationsbreite der Eigenschaften und Anwendungsbereiche, kann der Anteil an Verbindungen der Formel I in den erfindungsgemässen Gemischen in einem breiten Bereich variieren und etwa O,1 bis 1OO Gew.% betragen. Beispielsweise kann das Gemisch aus Verbindungen der Formel I bestehen. Anderseits werden z.B. chirale Dotierstoffe oft nur in relativ kleinen Mengen z.B. etwa O,1 bis 1O Gew.% eingesetzt. Im allgemeinen beträgt jedoch der Anteil an Verbindungen der Formel I in den erfindungsgemässen Gemischen etwa 1 - 60 Gew.%. Bevorzugt ist in der Regel ein Bereich von etwa 5 - 30 Gew.%.

Die erfindungsgemässen Gemische für nematische oder cholesterische Anwendungen enthalten neben einer oder mehreren Verbindungen der Formel I vorzugsweise eine oder mehrere Verbindungen aus der Gruppe der Verbindungen der allgemeinen Formeln 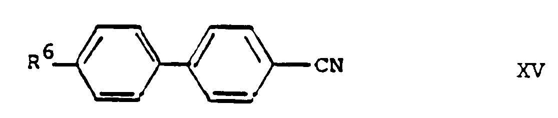 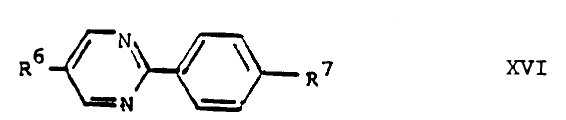 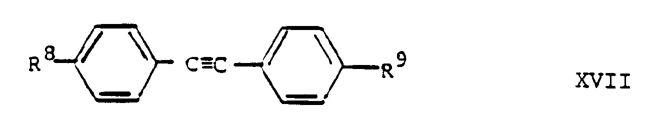 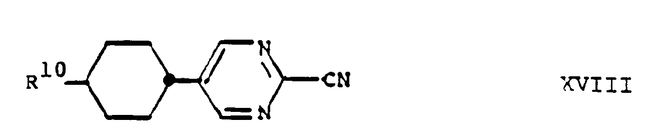 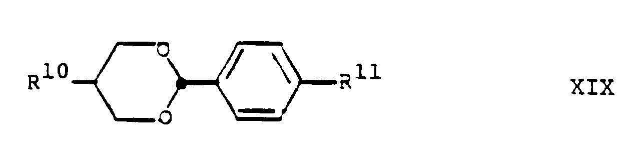 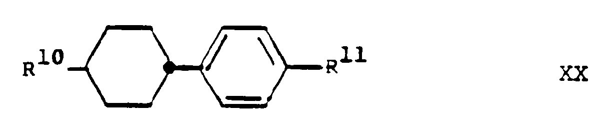 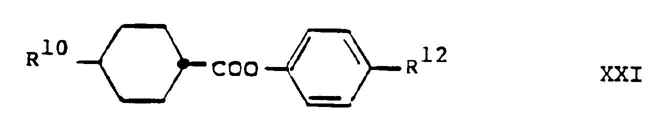 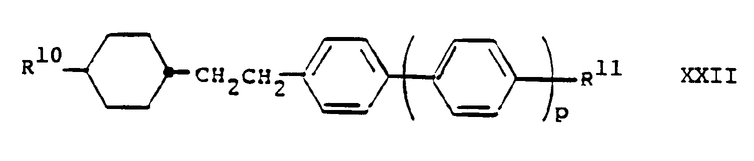 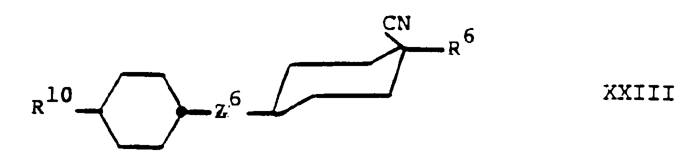 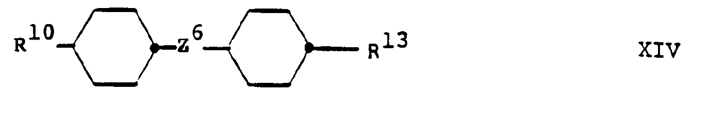 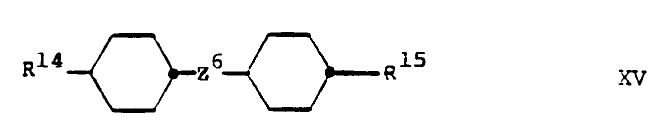 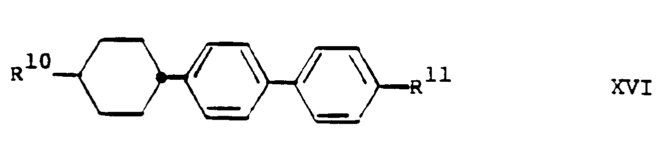 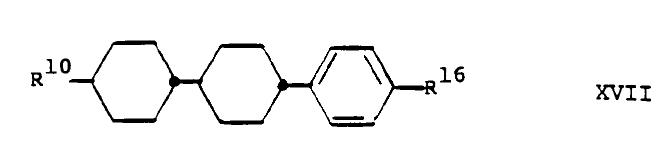 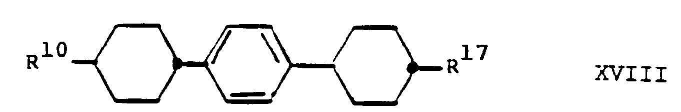 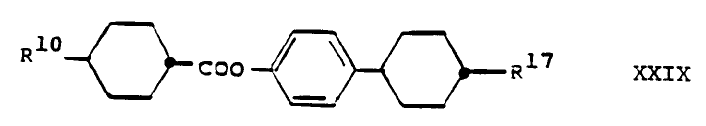 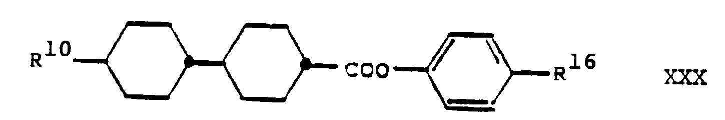 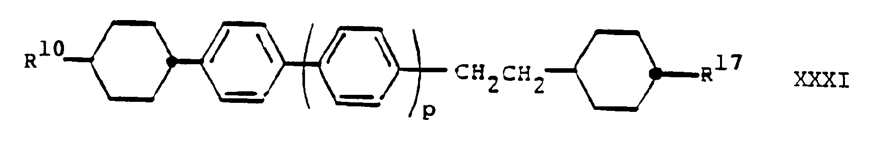 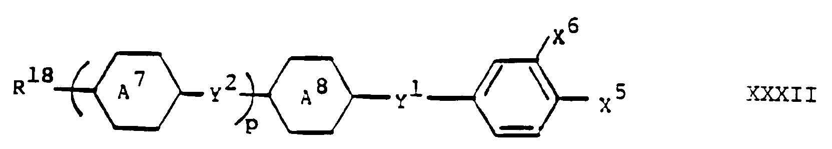
worin R⁶ Alkyl, 3E-Alkenyl oder 4-Alkenyl bedeutet; R⁷ Cyano oder Fluor darstellt; R⁸ und R⁹ Alkyl oder Alkoxy bezeichnen; R¹⁰ und R¹⁷ unabhängig voneinander Alkyl, 1E-Alkenyl, 3E-Alkenyl oder 4-Alkenyl bedeuten; R¹¹ Cyano, -NCS, Alkyl, 1E-Alkenyl, 3E-Alkenyl, 4-Alkenyl, Alkoxy, 2E-Alkenyloxy oder 3-Alkenyloxy bezeichnet; R¹² Alkoxy, 2E-Alkenyloxy oder 3-Alkenyloxy bedeutet; p für die Zahl O oder 1 steht; Z⁶ eine einfache Kovalenzbindung oder -CH₂CH₂- darstellt; R¹³ Cyano, Alkyl, 1E-Alkenyl, 3E-Alkenyl oder 4-Alkenyl bedeutet; R¹⁴ Alkyl, 1E-Alkenyl oder 4-Alkenyl bezeichnet; R¹⁵ Alkoxy, 2E-Alkenyloxy oder 3-Alkenyloxy darstellt; R¹⁶ Cyano, Alkyl, 1E-Alkenyl, 3E-Alkenyl, 4-Alkenyl, Alkoxy, 2E-Alkenyloxy oder 3-Alkenyloxy bezeichnet; X⁵ Fluor oder Chlor und X⁶ Wasserstoff, Fluor oder Chlor bezeichnen; R¹⁸ Alkyl, 3E-Alkenyl, 4-Alkenyl, Alkoxy, 2E-Alkenyloxy oder 3-Alkenyloxy bedeutet; eine der Gruppen Y¹ und Y² eine einfache Kovalenzbindung, -COO-, -OOC-, -CH₂CH₂-, -CH₂O- oder -OCH₂- und die andere der Gruppen Y¹ und Y² eine einfache Kovalenzbindung bedeutet; und die Ringe A⁷ und A⁸ unabhängig voneinander trans-1,4-Cyclohexylen, in welchem gegebenenfalls 2 nicht benachbarte CH₂-Gruppen durch Sauerstoff ersetzt sind, oder 1,4-Phenylen, in welchem gegebenenfalls 1 oder 2 CH₂-Gruppen durch Stickstoff ersetzt sind, darstellen.

Vorzugsweise besitzen die Reste R⁶ und R⁸-R¹⁸ höchstens je 12 Kohlenstoffatome, insbesondere höchstens je 7 Kohlenstoffatome.

Die erfindungsgemässen Gemische für smektische Anwendungen (insbesondere für getiltete smektische oder chiral getiltete smektische Phasen) enthalten neben einer oder mehreren Verbindungen der Formel I vorzugsweise eine oder mehrere Verbindungen aus der Gruppe der Verbindungen der allgemeinen Formeln 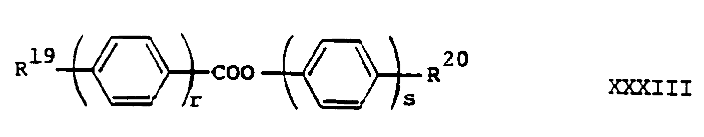 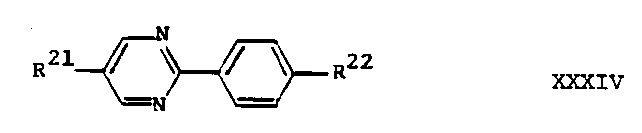 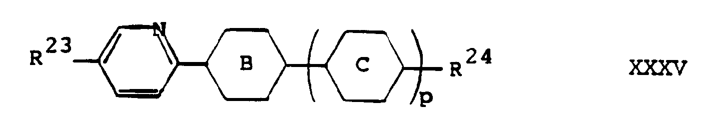 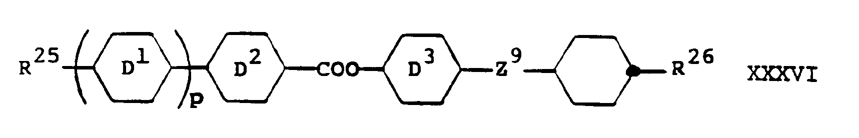 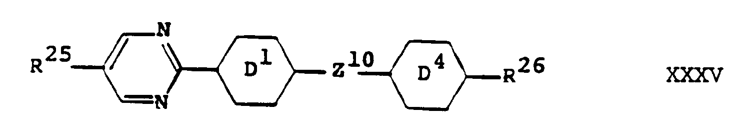 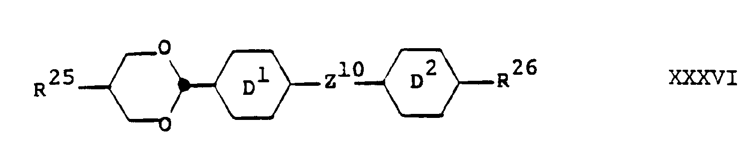 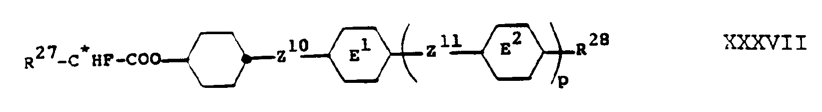 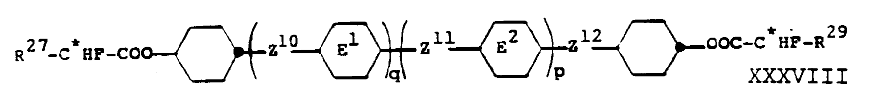
worin R¹⁹ und R²⁰ Alkyl, Alkoxy, Alkenyloxy, Alkanoyloxy oder Alkoxycarbonyl mit bis zu 18 Kohlenstoffatomen bezeichnen; r und s unabhängig voneinander 1 oder 2 bedeuten; R²¹ und R²² Alkyl, Alkoxy oder Alkenyloxy mit bis zu 18 Kohlenstoffatomen bezeichnen; Ring B unsubstituiertes oder mit Halogen und/oder Methyl substituiertes 1,4-Phenylen darstellt; Ring C trans-1,4-Cyclohexylen oder unsubstituiertes oder mit Halogen und/oder Methyl substituiertes 1,4-Phenylen darstellt; p und q unabhängig voneinander für die Zahl O oder 1 stehen; R²³ und R²⁴ unabhängig voneinander eine unsubstituierte oder mit Halogen substituierte C₁-C₁₈-Alkyl- oder C₂-C₁₈-Alkenylgruppe bezeichnen, in welcher gegebenenfalls eine oder zwei nicht benachbarte CH₂-Gruppen durch -O-, -COO- und /oder -OOC- ersetzt sind; die Ringe D¹, D² und D³ unabhängig voneinander unsubstituiertes oder mit Cyano, Halogen oder Niederalkyl substituiertes 1,4-Phenylen darstellen; Z⁹ eine einfache Kovalenzbindung, -CH₂CH₂-, -OCH₂-, -COO- oder -OOC- bezeichnet; R²⁵ und R²⁶ unabhängig voneinander eine unsubstituierte oder halogensubstituierte C₁-C₁₈-Alkyl- oder C₂-C₁₈-Alkenylgruppe bedeuten, in welcher gegebenenfalls eine oder zwei nicht benachbarte CH₂-Gruppen durch Sauerstoff ersetzt sind; Ring D⁴ trans-1,4-Cyclohexylen oder unsubstituiertes oder mit Cyano, Halogen oder Niederalkyl substituiertes 1,4-Phenylen darstellt; Z¹⁰, Z¹¹ und Z¹² unabhängig voneinander eine einfache Kovalenzbindung, -COO-, -OOC-, -CH₂CH₂-, -OCH₂- oder -CH₂O- bezeichnen; R²⁷ und R²⁹ unabhängig voneinander eine C₁-C₁₅-Alkyl- oder C₂-C₁₅-Alkenylgruppe bedeuten, in welcher gegebenenfalls eine CH₂-Gruppe durch Sauerstoff ersetzt ist; R²⁸ eine unsubstituierte oder mit Halogen substituierte C₁-C₁₅-Alkyl-oder C₂-C₁₅-Alkenylgruppe bezeichnet, in welcher gegebenenfalls eine CH₂-Gruppe durch Sauerstoff ersetzt ist und/oder gegebenenfalls eine CH₂-Gruppe durch eine Estergruppe -COO- oder -OOC- ersetzt ist; die Ringe E¹ und E² unabhängig voneinander unsubstituiertes oder mit Halogen, Cyano und/oder Methyl substituiertes 1,4-Phenylen darstellen, in welchem gegebenenfalls 1 oder 2 CH-Gruppen durch Stickstoff ersetzt sind; und C* ein chirales Kohlenstoffatom bezeichnet.

Die Erfindung betrifft ferner ein flüssigkristallines Gemisch mit mindestens 2 Komponenten, welches dadurch gekennzeichnet ist, dass mindestens eine Komponente eine Verbindung der Formel V ist, worin n für die Zahl O oder 1 steht und R¹ eine Gruppe R³-A⁴-Z³- bezeichnet oder n für die Zahl 1 steht und R¹ eine Gruppe R³ bezeichnet. Bevorzugt sind Gemische, welche eine oder mehrere der als bevorzugt genannten Verbindungen der Formel V enthalten.

Der Anteil an Verbindungen der Formel V im Gemisch kann in einem breiten Bereich variieren und beispielsweise etwa 1 - 50 Gew.% betragen. Bevorzugt ist im allgemeinen ein Bereich von etwa 5 - 30 Gew.%.

Die bicyclischen und tricyclischen Verbindungen der Formel V werden vorzugsweise als Komponenten nematischer oder cholesterischer Gemische verwendet. Vorzugsweise enthalten die Gemische neben einer oder mehreren bicyclischen oder tricyclischen Verbindungen der Formel V eine oder mehrere Verbindungen aus der Gruppe der Verbindungen der obigen Formeln I und XV - XXXII.

Die Herstellung der flüssigkristallinen Gemische und der elektro-optischen Vorrichtungen kann in an sich bekannter Weise erfolgen.

Die Erfindung wird durch die folgenden Beispiele weiter veranschaulicht. Die optischen Antipoden von chiralen Verbindungen besitzen jeweils die gleichen Phasenumwandlungstemperaturen und absolut gleiche Werte der Verdrillung aber mit entgegengesetzten Vorzeiche. Die zur Charakterisierung der Phasenumwandlungen verwendeten Abkürzungen stehen für folgende Bedeutungen:
- C: für kristallin
- S: für smektisch
- S_{A}, S_{B}, S_{C} etc.: für smektisch A, B, C etc.
- S_{C}*, S_{F}* etc.: für chiral smektisch C, F etc.
- N: für nematisch
- N*: für cholesterisch
- I: für isotrop.

### Beispiel 1

Ein Gemisch von 0,5 g (E)-3-(trans-4-Pentylcyclohexyl)-allylalkohol, 0,3 g 4-Methoxyphenol, 0,4 g Azodicarbonsäure-diäthylester, 0,6 g Triphenylphosphin und 25 ml absolutem Tetrahydrofuran wurde über Nacht bei Raumtemperatur gerührt und dann eingeengt. Der Rückstand wurde mit 50 ml heissem Hexan aufgeschlämmt und filtriert. Das Filtrat wurde eingeengt. Chromatographie des Rückstandes an Kieselgel mit Toluol/ Hexan (Vol. 1:1) und Umkristallisaiton aus Methanol ergab reinen 4-Methoxyphenyl-(E)-3-(trans-4-pentylcyclohexyl)allyläther mit Smp. (C-N) 45°C und Klp. (N-I) 46°C.

Der als Ausgangsmaterial verwendete (E)-3-(trans-4-Pentylcyclohexyl)allylalkohol wurde wie folgt hergestellt:

a) Ein Gemisch von 10 g trans-4-Pentylcyclohexancarboxaldehyd und 16 g Aethyl-diäthylphosphonoacetat (C₂H₅O)₂PCH₂COOC₂H₅ wurde bei Raumtemperatur tropfenweise mit einer Lösung von 15 g Kaliumcarbonat in 11 ml Wasser versetzt. Das Reaktionsgemisch wurde über Nacht bei Raumtemperatur gerührt und dann auf 500 ml Wasser gegossen und viermal mit je 50 ml Hexan extrahiert. Die vereinigten organischen Phasen wurden zweimal mit je 500 ml Wasser gewaschen, über Magnesiumsulfat getrocknet, filtriert und eingeengt. Chromatographie des Rückstandes an Kieselgel mit Toluol ergab schliesslich 13,5 g (E)-3-(trans-4-Pentylcyclohexyl)acrylsäureäthylester mit Sdp. 114 - 116°C/O,O7 mmHg.

b) Eine Lösung von 13,5 g (E)-3-(trans-4-Pentylcyclohexyl)acrylsäure-äthylester in 100 ml Dichlormethan wurde bei - 78°C und unter Stickstoffbegasung tropfenweise mit 100 ml einer 20%-igen Lösung von Diisobutylaluminiumhydrid in Hexan versetzt. Nach beendeter Zugabe wurde die leicht gelbe Lösung noch 1 Stunde gerührt und dann vorsichtig mit 10 ml 25%-iger Salzsäure versetzt. Das Reaktionsgemisch wurde auf 100 ml Wasser gegossen und die organische Phase abgetrennt. Die wässrige Phase wurde zweimal mit je 100 ml Dichlormethan nachextrahiert. Die vereinigten organischen Phasen wurden mit 500 ml Wasser, 500 ml konzentrierter Kaliumhydrogencarbonat-Lösung und nochmals mit 500 ml Wasser gewaschen und anschliessend über Magnesiumsulfat getrocknet, filtriert und eingeengt. Destillation des Rückstandes ergab 10 g (E)-3-(trans-4-Pentylcyclohexyl)allylalkohol mit Sdp. 174 - 175°C/15 mmHg.

In analoger Weise können folgende Verbindungen hergestellt werden:
4-Aethoxyphenyl-(E)-3-(trans-4-pentylcyclohexyl)allyl-äther, Smp. (C-I) 62°C, Klp. (N-I) 59°C;
4-Propyloxyphenyl-(E)-3-(trans-4-pentylcyclohexyl)allyl-äther, Smp. (C-I) 72°C, Klp. (N-I) 5O°C;
4-Butyloxyphenyl-(E)-3-(trans-4-pentylcyclohexyl)allyl-äther, Smp. (C-I) 59°C, S_{A}-N 43°C, Klp. (N-I) 61°C;
4-Pentyloxyphenyl-(E)-3-(trans-4-pentylcyclohexyl)allyl-äther, Smp. (C-S_{A}) 41°C, S_{A}-N 46°C, Klp. (N-I) 54°C;
4-Hexyloxyphenyl-(E)-3-(trans-4-pentylcyclohexyl)allyl-äther, Smp. (C-S_{A}) 50°C, S_{A}-N 55°C, Klp. (N-I) 60°C;
4-Heptyloxyphenyl-(E)-3-(trans-4-pentylcyclohexyl)allyl-äther, Smp. (C-S_{A}) 42°C, S_{A}-N 57°C, Klp. (N-I) 58°C;
4-Octyloxyphenyl-(E)-3-(trans-4-pentylcyclohexyl)allyl-äther;
4-Nonyloxyphenyl-(E)-3-(trans-4-pentylcyclohexyl)allyl-äther;
4-Decyloxyphenyl-(E)-3-(trans-4-pentylcyclohexyl)allyl-äther;
4-Cyanophenyl-(E)-3-(trans-4-methylcyclohexyl)allyl-äther;
4-Cyanophenyl-(E)-3-(trans-4-aethylcyclohexyl)allyl-äther;
4-Cyanophenyl-(E)-3-(trans-4-propylcyclohexyl)allyl-äther;
4-Cyanophenyl-(E)-3-(trans-4-butylcyclohexyl)allyl-äther;
4-Cyanophenyl-(E)-3-(trans-4-pentylcyclohexyl)allyl-äther, Smp. (C-I) 64°C, Klp. (N-I) 43°C;
4-Cyanophenyl-(E)-3-(trans-4-hexylcyclohexyl)allyl-äther;
4-Cyanophenyl-(E)-3-(trans-4-heptylcyclohexyl)allyl-äther;
4-Cyanophenyl-(E)-3-(trans-4-octylcyclohexyl)allyl-äther;
4-Fluorphenyl-(E)-3-(trans-4-propylcyclohexyl)allyl-äther;
4-Fluorphenyl-(E)-3-(trans-4-pentylcyclohexyl)allyl-äther, Smp. (C-I) 23°C;
3,4-Difluorphenyl-(E)-3-(trans-4-propylcyclohexyl)allyl-äther;
3,4-Difluorphenyl-(E)-3-(trans-4-pentylcyclohexyl)allyl-äther, Smp. (C-I) 11°C;
2,3-Difluor-4-methoxyphenyl-(E)-3-(trans-4-pentylcyclohexyl)allyl-äther;
2,3-Difluor-4-aethoxyphenyl-(E)-3-(trans-4-pentylcyclohexyl)allyl-äther, Smp. (C-I) 23°C, Klp. (N-I) 5°C;
2,3-Difluor-4-propyloxyphenyl-(E)-3-(trans-4-pentylcyclohexyl)allyl-äther;
2,3-Difluor-4-butyloxyphenyl-(E)-3-(trans-4-pentylcyclohexyl)allyl-äther;
4-Chlorphenyl-(E)-3-(trans-4-pentylcyclohexyl)allyl-äther, Smp. (C-I) 55°C;
4-Bromphenyl-(E)-3-(trans-4-pentylcyclohexyl)allyl-äther, Smp. (C-I) 65°C;
4-Iodphenyl-(E)-3-(trans-4-pentylcyclohexyl)allyl-äther, Smp. (C-I) 66°C;
2,3-Dicyano-4-pentylphenyl-(E)-3-(trans-4-propylcyclohexyl)allyl-äther;
2,3-Dicyano-4-pentylphenyl-(E)-3-(trans-4-pentylcyclohexyl)allyl-äther;
2,3-Dicyano-4-pentylphenyl-(E)-3-(trans-4-heptylcyclohevyl)allyl-äther;
2-Fluor-4-cyanophenyl-(E)-3-(trans-4-propylcyclohexyl)allyl-äther;
2-Fluor-4-cyanophenyl-(E)-3-(trans-4-pentylcyclohexyl)allyl-äther;
2-Fluor-4-cyanophenyl-(E)-3-(trans-4-heptylcyclohexyl)allyl-äther;
3-Fluor-4-cyanophenyl-(E)-3-(trans-4-propylcyclohexyl)allyl-äther;
3-Fluor-4-cyanophenyl-(E)-3-(trans-4-pentylcyclohexyl)allyl-äther, Smp. (C-I) 51°C;
4-(trans-4-Propylcyclohexyl)phenyl-(E)-3-(trans-4-propylcyclohexyl)allyl-äther;
4-(trans-4-Pentylcyclohexyl)phenyl-(E)-3-(trans-4-propylcyclohexyl)allyl-äther;
4-(trans-4-Heptylcyclohexyl)phenyl-(E)-3-(trans-4-propylcyclohexyl)allyl-äther;
4-(trans-4-Propylcyclohexyl)phenyl-(E)-3-(trans-4-pentylcyclohexyl)allyl-äther;
4-(trans-4-Pentylcyclohexyl)phenyl-(E)-3-(trans-4-pentylcyclohexyl)allyl-äther, Smp. (C-S_{B}) 56°C, S_{B}-S_{A} 109°C, S_{A}-N 113°C, Klp. (N-I) 136°C;
4-(trans-4-Heptylcyclohexyl)phenyl-(E)-3-(trans-4-pentylcyclohexyl)allyl-äther;
4-(trans-4-Propylcyclohexyl)phenyl-(E)-3-(trans-4-heptylcyclohexyl)allyl-äther;
4-(trans-4-Pentylcyclohexyl)phenyl-(E)-3-(trans-4-heptylcyclohexyl)allyl-äther;
4-(trans-4-Heptylcyclohexyl)phenyl-(E)-3-(trans-4-heptylcyclohexyl)allyl-äther;
4-[2-(trans-4-Propylcyclohexyl)äthyl]phenyl-(E)-3-(trans-4-propylcyclohexyl)allyl-äther;
4-[2-(trans-4-Pentylcyclohexyl)äthyl]phenyl-(E)-3-(trans-4-propylcyclohexyl)allyl-äther;
4-[2-(trans-4-Heptylcyclohexyl)äthyl]phenyl-(E)-3-(trans-4-propylcyclohexyl)allyl-äther;
4-[2-(trans-4-Propylcyclohexyl)äthyl]phenyl-(E)-3-(trans-4-pentylcyclohexyl)allyl-äther;
4-[2-(trans-4-Pentylcyclohexyl)äthyl]phenyl-(E)-3-(trans-4-pentylcyclohexyl)allyl-äther, Smp. (C-S_{B}) 88°C, S_{B}-S_{A} 103°C, S_{A}-N 110°C, Klp. (N-I) 123°C;
4-[2-(trans-4-Heptylcyclohexyl)äthyl]phenyl-(E)-3-(trans-4-pentylcyclohexyl)allyl-äther;
4-[2-(trans-4-Propylcyclohexyl)äthyl]phenyl-(E)-3-(trans-4-heptylcyclohexyl)allyl-äther;
4-[2-(trans-4-Pentylcyclohexyl)äthyl]phenyl-(E)-3-(trans-4-heptylcyclohexyl)allyl-äther;
4-[2-(trans-4-Heptylcyclohexyl)äthyl]phenyl-(E)-3-(trans-4-heptylcyclohexyl)allyl-äther;
4-[(trans-4-Propylcyclohexyl)methoxy]phenyl-(E)-3-(trans-4-pentylcyclohexyl)allyl-äther;
4-[(trans-4-Pentylcyclohexyl)methoxy]phenyl-(E)-3-(trans-4-pentylcyclohexyl)allyl-äther, Smp. (C-S_{B}) 84°C, S_{B}-S_{A} 101°C, Klp. (S_{A}-I) 116°C;
4-[3-(trans-4-Propylcyclohexyl)-1-propyloxy-(E)-3-(trans-4-pentylcyclohexyl)allyl-äther;
4-[3-(trans-4-Pentylcyclohexyl)-1-propyloxy-(E)-3-(trans-4-pentylcyclohexyl)allyl-äther, Smp. (C-S_{A}) 115°C, S_{A}-N 116°C, Klp. (N-I) 120°C;
4-[3-(trans-4-Heptylcyclohexyl)-1-propyloxy-(E)-3-(trans-4-pentylcyclohexyl)allyl-äther;
4'-(trans-4-Propylcyclohexyl)-4-biphenylyl-(E)-3-)trans-4-propylcyclohexyl)allyl-äther;
4'-(trans-4-Propylcyclohexyl)-4-biphenylyl-(E)-3-(trans-4-pentylcyclohexyl)allyl-äther;
4'-(trans-4-Pentylcyclohexyl)-4-biphenylyl-(E)-3-(trans-4-propylcyclohexyl)allyl-äther;
4'-(trans-4-Pentylcyclohexyl)-4-biphenylyl-(E)-3-(trans-4-pentylcyclohexyl)allyl-äther;
4'-Cyano-4-biphenylyl-(E)-3-(trans-4-propylcyclohexyl)allyl-äther;
4'-Cyano-4-biphenylyl-(E)-3-(trans-4-pentylcyclohexyl)allyl-äther;
4'-Cyano-4-biphenylyl-(E)-3-(trans-4-heptylcyclohexyl)allyl-äther;
4'-Fluor-4-biphenylyl-(E)-3-(trans-4-propylcyclohexyl)allyl-äther;
4'-Fluor-4-biphenylyl-(E)-3-(trans-4-pentylcyclohexyl)allyl-äther;
4'-Fluor-4-biphenylyl-(E)-3-(trans-4-heptylcyclohexyl)allyl-äther;
3',4'-Difluor-4-biphenylyl-(E)-3-(trans-4-propylcyclohexyl)allyl-äther;
3',4'-Difluor-4-biphenylyl-(E)-3-(trans-4-pentylcyclohexyl)allyl-äther;
3',4'-Difluor-4-biphenylyl-(E)-3-(trans-4-heptylcyclohexyl)allyl-äther;
Aethyl-(R)-2-(4'-[(E)-3-(trans-4-pentylcyclohexyl)allyloxy]-4-biphenylyloxy)propionat, Smp. (C-Ch) 77°C, S_{A}-Ch 76°C, Klp. (Ch-I) 83°C;
5-Pentyl-2-(4-[(E)-3-(trans-4-pentylcyclohexyl)allyloxy]phenyl)pyrimidin, Smp. (C-N) 77°C, Klp. (N-I) 144°C;
5-Hexyl-2-(4-[(E)-3-(trans-4-pentylcyclohexyl)allyloxy]phenyl)pyrimidin, Smp. (C-N) 83°C, Klp. (N-I) 139°C;
5-Heptyl-2-(4-[(E)-3-(trans-4-pentylcyclohexyl)allyloxy]phenyl)pyrimidin, Smp. (C-N) 91°C, Klp. (N-I) 141°C;
5-Octyl-2-(4-[(E)-3-(trans-4-pentylcyclohexyl)allyloxy]phenyl)pyrimidin, Smp. (C-S_{C}) 87°C, S_{C}-N 88°C, Klp. (N-I) 138°C;
5-Nonyl-2-(4-[(E)-3-(trans-4-pentylcyclohexyl)allyloxy]phenyl)pyrimidin, Smp. (C-S_{C}) 79°C, S_{C}-N 102°C, Klp. (N-I) 138°C;
5-Decyl-2-(4-[(E)-3-(trans-4-pentylcyclohexyl)allyloxy]phenyl)pyrimidin;
5-Octyl-2-(4-[(E)-3-(trans-4-pentylcyclohexyl)allyloxy]phenyl)pyridin;
5-Nonyl-2-(4-[(E)-3-(trans-4-pentylcyclohexyl)allyloxy]phenyl)pyridin;
5-Decyl-2-(4-[(E)-3-(trans-4-pentylcyclohexyl)allyloxy]phenyl)pyridin, Smp. (C-S) -35°C, S-S 128°C, S-S_{C} 130°C, Klp. (S_{C}-I) 145°C;
3,4-Difluorphenyl-(E)-3-[trans-4-(trans-4-propylcyclohexyl)cyclohexyl)allyl-äther, Smp. (C-N) 48°C, Klp. (N-I) 101°C;
3,4-Difluorphenyl-(E)-3-[trans-4-(trans-4-butylcyclohexyl)cyclohexyl)allyl-äther;
3,4-Difluorphenyl-(E)-3-[trans-4-(trans-4-pentylcyclohexyl)cyclohexyl)allyl-äther, Smp. (C-N) 52°C, Klp. (N-I) 103°C;
4-Fluorphenyl-(E)-3-[trans-4-(trans-4-propylcyclohexyl)cyclohexyl)allyl-äther, Smp. (C-N) 75°C, Klp. (N-I) 127°C;
4-Fluorphenyl-(E)-3-[trans-4-(trans-4-butylcyclohexyl)cyclohexyl)allyl-äther;
4-Fluorphenyl-(E)-3-[trans-4-(trans-4-pentylcyclohexyl)cyclohexyl)allyl-äther, Smp. (C-N) 68°C, S_{B}-N 60°C, Klp. (N-I) 131°C;
4-Cyano-2-fluorphenyl-(E)-3-[trans-4-(trans-3-propylcyclohexyl)cyclohexyl]allyl-äther;
4-Cyano-2-fluorphenyl-(E)-3-[trans-4-(trans-3-pentylcyclohexyl)cyclohexyl]allyl-äther;
4-Cyano-2-fluorphenyl-(E)-3-[trans-4-(trans-3-heptylcyclohexyl)cyclohexyl]allyl-äther;
4-Cyano-3-fluorphenyl-(E)-3-[trans-4-(trans-3-propylcyclohexyl)cyclohexyl]allyl-äther, Smp. (C-N) 93°C, Klp. (N-I) 138°C;
4-Cyano-3-fluorphenyl-(E)-3-[trans-4-(trans-3-pentylcyclohexyl)cyclohexyl]allyl-äther, Smp. (C-N) 99°C, Klp. (N-I) 141°C;
4-Cyanophenyl-(E)-3-[trans-4-(trans-4-propylcyclohexyl)cyclohexyl)allyl-äther, Smp. (C-N) 96°C, Klp. (N-I) 166°C;
4-Cyanophenyl-(E)-3-[trans-4-(trans-4-pentylcyclohexyl)cyclohexyl)allyl-äther, Smp. (C-N) 102°C, Klp. (N-I) 165°C;
2,3-Difluor-4-methoxyphenyl-(E)-3-[trans-4-(trans-4-propylcyclohexyl)cyclohexyl]allyl-äther;
2,3-Difluor-4-aethoxyphenyl-(E)-3-[trans-4-(trans-4-propylcyclohexyl)cyclohexyl]allyl-äther, Smp. (C-N) 65°C, Klp. (N-I) 136°C;
2,3-Difluor-4-propyloxyphenyl-(E)-3-[trans-4-(trans-4-propylcyclohexyl)cyclohexyl]allyl-äther;
2,3-Difluor-4-butyloxyphenyl-(E)-3-[trans-4-(trans-4-propylcyclohexyl)cyclohexyl]allyl-äther, Smp. (C-N) 54°C, Klp. (N-I) 127°C;
2,3-Difluor-4-pentyloxyphenyl-(E)-3-[trans-4-(trans-4-propylcyclohexyl)cyclohexyl]allyl-äther;
2,3-Difluor-4-methoxyphenyl-(E)-3-[trans-4-(trans-4-pentylcyclohexyl)cyclohexyl]allyl-äther;
2,3-Difluor-4-aethoxyphenyl-(E)-3-[trans-4-(trans-4-pentylcyclohexyl)cyclohexyl]allyl-äther, Smp. (C-N) 51°C, Klp. (N-I) 140°C;
2,3-Difluor-4-propyloxyphenyl-(E)-3-[trans-4-(trans-4-pentylcyclohexyl)cyclohexyl]allyl-äther;
2,3-Difluor-4-butyloxyphenyl-(E)-3-[trans-4-(trans-4-pentylcyclohexyl)cyclohexyl]allyl-äther;
2,3-Difluor-4-pentyloxyphenyl-(E)-3-[trans-4-(trans-4-pentylcyclohexyl)cyclohexyl]allyl-äther;
2,3-Dicyano-4-pentylphenyl-(E)-3-[trans-4-(trans-4-propylcyclohexyl)cyclohexyl]allyl-äther;
2,3-Dicyano-4-pentylphenyl-(E)-3-[trans-4-(trans-4-pentylcyclohexyl)cyclohexyl]allyl-äther;
2,3-Dicyano-4-pentylphenyl-(E)-3-[trans-4-(trans-4-heptylcyclohexyl)cyclohexyl]allyl-äther;
4-(trans-4-Propylcyclohexyl)phenyl-(E)-3-[trans-4-(trans-4-propylcyclohexyl)cyclohexyl]allyl-äther;
4-(trans-4-Propylcyclohexyl)phenyl-(E)-3-[trans-4-(trans-4-pentylcyclohexyl)cyclohexyl]allyl-äther;
4-(trans-4-Pentylcyclohexyl)phenyl-(E)-3-[trans-4-(trans-4-propylcyclohexyl)cyclohexyl]allyl-äther;
4-(trans-4-Pentylcyclohexyl)phenyl-(E)-3-[trans-4-(trans-4-pentylcyclohexyl)cyclohexyl]allyl-äther;
4-[(4aαH,8aβH)-Decahydro-6β-pentyl-2α-naphthyl]phenyl-(E)-3-(trans-4-pentylcyclohexyl)allyl-äther, Smp. (C-S_{A}) 96°C, S_{A}-N 127°C, Klp. (N-I) 172°C.

### Beispiel 2

2,O g 2,3-Difluor-4-dodecyloxybenzoesäure, 1,8 g 4-[(E)-3-(trans-4-Pentylcyclohexyl)allyloxy]phenol und O,1 g 4-(Dimethylamino)pyridin wurden in 5O ml Dichlormethan gelöst und die Lösung innert 1O Minuten unter Rühren portionenweise mit 1,4 g N,N'-Dicyclohexylcarbodiimid versetzt. Das Gemisch wurde über Nacht bei Raumtemperatur gerührt und dann filtriert. Das Filtrat wurde mit Dichlormethan verdünnt, zweimal mit je 5O ml gesättigter Natriumcarbonat-Lösung und dann mit Wasser gewaschen, über Magnesiumsulfat getrocknet und eingeengt. Das erhaltene Rohprodukt wurde an Kieselgel mit Toluol chromatographisch gereinigt. Der erhaltene 2,3-Difluor-4-dodecyloxybenzoesäure-4-[(E)-3-(trans-4-pentylcyclohexyl)allyloxy]phenylester wurde aus Aethanol umkristallisiert; Smp. (C-S_{C}) 73°C, S_{C}-N 124°C, Klp. (N-I) 137°C.

Das als Ausgangsmaterial verwendete 4-[(E)-3-(trans-4-Pentylcyclohexyl)allyloxy]phenol wurde wie folgt hergestellt:

1O g (E)-3-(trans-4-Pentylcyclohexyl)allylalkohol, 26 g Hydrochinon, 8 g Azodicarbonsäurediäthylester, 12 g Triphenylphosphin und 5OO ml Tetrahydrofuran wurden in analoger Weise zu Beispiel 1 umgesetzt. Dies ergab 16 g 4-[(E)-3-(trans-4-Pentylcyclohexyl)allyloxy]phenol, Smp. 97 - 98°C.

In analoger Weise können folgende Verbindungen hergestellt werden:
2,3-Difluor-4-heptyloxybenzoesäure-4-[(E)-3-(trans-4-pentylcyclohexyl)allyloxy]phenylester, Smp. (C-S_{C}) 66°C, S_{C}-N 95°C, Klp. (N-I) 144°C;
2,3-Difluor-4-octyloxybenzoesäure-4-[(E)-3-(trans-4-pentylcyclohexyl)allyloxy]phenylester, Smp. (C-S_{C}) 76°C, S_{C}-N 1O6°C, Klp. (N-I) 143°C;
2,3-Difluor-4-nonyloxybenzoesäure-4-[(E)-3-(trans-4-pentylcyclohexyl)allyloxy]phenylester, Smp. (C-S_{C}) 74°C, S_{C}-N 114°C, Klp. (N-I) 141°C;
2,3-Difluor-4-decyloxybenzoesäure-4-[(E)-3-(trans-4-pentylcyclohexyl)allyloxy]phenylester, Smp. (C-S_{C}) 8O°C, S_{C}-N 119°C, Klp. (N-I) 14O°C;
2,3-Difluor-4-undecyloxybenzoesäure-4-[(E)-3-(trans-4-pentylcyclohexyl)allyloxy]phenylester, Smp. (C-S_{C}) 77°C, S_{C}-N 121°C, Klp. (N-I) 137°C;
4-Methoxybenzoesäure-4-[(E)-3-(trans-4-pentylcyclohexyl)allyloxy]phenylester;
4-Aethoxybenzoesäure-4-[(E)-3-(trans-4-pentylcyclohexyl)allyloxy]phenylester;
4-Propyloxybenzoesäure-4-[(E)-3-(trans-4-pentylcyclohexyl)allyloxy]phenylester;
4-Butyloxybenzoesäure-4-[(E)-3-(trans-4-pentylcyclohexyl)allyloxy]phenylester;
4-Pentyloxybenzoesäure-4-[(E)-3-(trans-4-pentylcyclohexyl)allyloxy]phenylester;
4-Hexyloxybenzoesäure-4-[(E)-3-(trans-4-pentylcyclohexyl)allyloxy]phenylester;
4-Heptyloxybenzoesäure-4-[(E)-3-(trans-4-pentylcyclohexyl)allyloxy]phenylester;
4-Octyloxybenzoesäure-4-[(E)-3-(trans-4-pentylcyclohexyl)allyloxy]phenylester;
4-Nonyloxybenzoesäure-4-[(E)-3-(trans-4-pentylcyclohexyl)allyloxy]phenylester;
4-Decyloxybenzoesäure-4-[(E)-3-(trans-4-pentylcyclohexyl)allyloxy]phenylester;
trans-4-Methylcyclohexancarbonsäure-4-[(E)-3-(trans-4-pentylcyclohexyl)allyloxy]phenylester;
trans-4-Aethylcyclohexancarbonsäure-4-[(E)-3-(trans-4-pentylcyclohexyl)allyloxy]phenylester;
trans-4-Propylcyclohexancarbonsäure-4-[(E)-3-(trans-4-pentylcyclohexyl)allyloxy]phenylester;
trans-4-Butylcyclohexancarbonsäure-4-[(E)-3-(trans-4-pentylcyclohexyl)allyloxy]phenylester;
trans-4-Pentylcyclohexancarbonsäure-4-[(E)-3-(trans-4-pentylcyclohexyl)allyloxy]phenylester, Smp. (C-S_{A}) 119°C, S_{A}-N 123°C, Klp. (N-I) 156°C.

### Beispiel 3

Eine Lösung von O,5 g 4-[(E)-3-(trans-4-Pentylcyclohexyl)allyloxy]benzaldehyd und O,3 g 2-Pentyl-1,3-propandiol in 5O ml Toluol wurde mit O,O5 g 4-Toluolsulfonsäure versetzt. Das Gemisch wurde 2,5 Stunden zum Sieden erhitzt, wobei das entstandene Wasser gleichzeitig abdestilliert wurde. Dann wurden 4 Tropen Tiäthylamin zum Reaktionsgemisch zugegeben. Nach dem Erkalten wurde das Gemisch mit 2O ml 1N Natriumhydrogencarbonat-Lösung und zweimal mit je 2O ml Wasser gewaschen, über Natriumsulfat getrocknet und eingeengt. Chromatographie des Rückstandes an Kieselgel mit Toluol und Umkristallisation aus Aethylacetat ergab O,15 g trans-5-Pentyl-2-(4-[(E)-3-(trans-4-pentylcyclohexyl)allyloxy]phenyl)-1,3-dioxan.

Der als Ausgangsmaterial verwendete 4-[(E)-3-(trans-4-Pentylcyclohexyl)allyloxy]benzaldehyd wurde wie folgt hergestellt:

2,9 g 4-Hydroxybenzaldehyd, 5,O g (E)-3-(trans-4-Pentylcyclohexyl)allylalkohol, 4,1 g Azodicarbonsäurediäthylester, 6,2 g Triphenylphosphin und 1OO ml Tetrahydrofuran wurden in analoger Weise zu Beispiel 1 zu 4,5 g 4-[(E)-3-(trans-4-Pentylcyclohexyl)allyloxy]benzaldehyd (Smp. 54 - 55°C) umgesetzt.

In analoger Weise können folgende Verbindungen hergestellt werden:
trans-5-Methyl-2-(4-[(E)-3-(trans-4-propylcyclohexyl)allyloxy]phenyl)-1,3-dioxan;
trans-5-Aethyl-2-(4-[(E)-3-(trans-4-propylcyclohexyl)allyloxy]phenyl)-1,3-dioxan;
trans-5-Propyl-2-(4-[(E)-3-(trans-4-propylcyclohexyl)allyloxy]phenyl)-1,3-dioxan;
trans-5-Butyl-2-(4-[(E)-3-(trans-4-propylcyclohexyl)allyloxy]phenyl)-1,3-dioxan;
trans-5-Pentyl-2-(4-[(E)-3-(trans-4-propylcyclohexyl)allyloxy]phenyl)-1,3-dioxan;
trans-5-Hexyl-2-(4-[(E)-3-(trans-4-propylcyclohexyl)allyloxy]phenyl)-1,3-dioxan;
trans-5-Heptyl-2-(4-[(E)-3-(trans-4-propylcyclohexyl)allyloxy]phenyl)-1,3-dioxan;
trans-5-Methyl-2-(4-[(E)-3-(trans-4-pentylcyclohexyl)allyloxy]phenyl)-1,3-dioxan;
trans-5-Aethyl-2-(4-[(E)-3-(trans-4-pentylcyclohexyl)allyloxy]phenyl)-1,3-dioxan;
trans-5-Propyl-2-(4-[(E)-3-(trans-4-pentylcyclohexyl)allyloxy]phenyl)-1,3-dioxan;
trans-5-Butyl-2-(4-[(E)-3-(trans-4-pentylcyclohexyl)allyloxy]phenyl)-1,3-dioxan;
trans-5-Hexyl-2-(4-[(E)-3-(trans-4-pentylcyclohexyl)allyloxy]phenyl)-1,3-dioxan;
trans-5-Heptyl-2-(4-[(E)-3-(trans-4-pentylcyclohexyl)allyloxy]phenyl)-1,3-dioxan.

### Beispiel 4

1,4 g 4-Hydroxy-2-fluorbenzonitril, 3,3, g 4-[(E)-3-(trans-4-Pentylcyclohexyl)allyloxy]benzoesäure, 2,5 g N,N'-Dicyclohexylcarbodiimid, O,1 g 4-(Dimethylamino)pyridin und 1OO ml Dichlormethan wurden in analoger Weise zu Beispiel 2 umgesetzt. Dies ergab 1,5 g 4-[(E)-3-(trans-4-Pentylcyclohexyl)allyloxy]benzoesäure-4-cyano-3-fluorphenylester; Smp. (C-S_{A}) 54°C, S_{A}-N 68°C, Klp. (N-I) 145°C.

Die als Ausgangsmaterial verwendete 4-[(E)-3-(trans-4-Pentylcyclohexyl)allyloxy]benzoesäure wurde wie folgt hergestellt:

Eine Lösung von 4 g 4-[(E)-3-(trans-4-Pentylcyclohexyl)allyloxy]benzaldehyd (hergestellt nach Beispiel 3) in 1OO ml Aceton wurde tropfenweise mit 1O ml Jones' Reagenz versetzt. Das Gemisch wurde 1 Stunde bei Raumtemperatur gerührt und dann auf 1OO ml Wasser gegossen. Der dabei entstandene Niederschlag wurde abfiltriert, portionenweise mit Wasser gewaschen und am Vakuum getrocknet. Das Rohprodukt wurde aus Aethanol umkristallisiert und ergab 2,5 g reine 4-[(E)-3-(trans-4-Pentylcyclohexyl)allyloxy]benzoesäure; Smp. 214-215°C.

In analoger Weise können folgende Verbindungen hergestellt werden:
4-[(E)-3-(trans-4-Propylcyclohexyl)allyloxy]benzoesäure-4-cyano-3-fluorphenylester;
4-[(E)-3-(trans-4-Pentylcyclohexyl)allyloxy]benzoesäure-3,4-difluorphenylester, Smp. (C-N) 93°C, S_{A}-N 88°C, Klp. (N-I) 111°C;
4-[(E)-3-(trans-4-Propylcyclohexyl)allyloxy]benzoesäure-4-cyano-2-fluorphenylester;
4-[(E)-3-(trans-4-Pentylcyclohexyl)allyloxy]benzoesäure-4-cyano-2-fluorphenylester;
4-[(E)-3-(trans-4-Heptylcyclohexyl)allyloxy]benzoesäure-4-cyano-2-fluorphenylester;
4-[(E)-3-(trans-4-Pentylcyclohexyl)allyloxy]benzoesäure-4-fluorphenylester, Smp. (C-N) 105°C, S_{A}-N 102°C, Klp. (N-I) 132°C;
4-[(E)-3-(trans-4-Pentylcyclohexyl)allyloxy]benzoesäure-4-chlorphenylester, Smp. (C-S_{A}) 131°C, S_{A}-N 135°C, Klp. (N-I) 155°C;
4-[(E)-3-(trans-4-Pentylcyclohexyl)allyloxy]benzoesäure-4-bromphenylester, Smp. (C-S_{A}) 136°C, S_{A}-N 145°C, Klp. (N-I) 157°C;
4-[(E)-3-(trans-4-Pentylcyclohexyl)allyloxy]benzoesäure-4-jodphenylester, Smp. (C-S_{A}) 131°C, S_{A}-N 143°C, Klp. (N-I) 152°C;
4-[(E)-3-(trans-4-Pentylcyclohexyl)allyloxy]benzoesäure-4-cyanophenylester, Smp. (C-N) 79°C, Klp. (N-I) 170°C;
4-[(E)-3-(trans-4-Pentylcyclohexyl)allyloxy]benzoesäure-4-methylphenylester;
4-[(E)-3-(trans-4-Pentylcyclohexyl)allyloxy]benzoesäure-4-aethylphenylester;
4-[(E)-3-(trans-4-Pentylcyclohexyl)allyloxy]benzoesäure-4-propylphenylester;
4-[(E)-3-(trans-4-Pentylcyclohexyl)allyloxy]benzoesäure-4-butylphenylester;
4-[(E)-3-(trans-4-Pentylcyclohexyl)allyloxy]benzoesäure-4-pentylphenylester;
4-[(E)-3-(trans-4-Pentylcyclohexyl)allyloxy]benzoesäure-4-methylester;
4-[(E)-3-(trans-4-Pentylcyclohexyl)allyloxy]benzoesäure-4-aethylester;
4-[(E)-3-(trans-4-Pentylcyclohexyl)allyloxy]benzoesäure-4-propylester;
4-[(E)-3-(trans-4-Pentylcyclohexyl)allyloxy]benzoesäure-4-butylester;
4-[(E)-3-(trans-4-Pentylcyclohexyl)allyloxy]benzoesäure-4-pentylester.

### Beispiel 5

Ein Gemisch von O,1 g Natriumhydrid und 25 ml Tetrahydrofuran wurde unter Stickstoffbegasung mit O,5 g trans-4-Propylcyclohexanol versetzt, noch 2 Stunden gerührt, dann mit 1,O g (E)-3-(trans-4-Pentylcyclohexyl)allylbromid versetzt und anschliessend über Nacht auf 7O°C erwärmt. Danach wurde das Reaktionsgemisch mit 5OO ml Wasser versetzt und viermal mit je 5O ml Hexan extrahiert. Die vereinigten organischen Phasen wurden zweimal mit je 5OO ml Wasser gewaschen, über Magnesiumsulfat getrocknet, filtriert und dann eingeengt. Der Rückstand wurde an Kieselgel mit Toluol chromatographisch gereinigt. Der erhaltene trans-4-Propylcyclohexyl-(E)-3-(trans-4-pentylcyclohexyl)allyl-äther wurde aus Aceton bei -25°C umkristallisiert. Dies ergab 1,2 g reines Produkt mit Smp. (C-I) 26°C.

Das als Ausgangsmaterial verwendete (E)-3-(trans-4-Pentylcyclohexyl)allylbromid wurde wie folgt hergestellt:

Ein Gemisch von 9 g (E)-3-(trans-4-Pentylcyclohexyl)allylalkohol, 12,3 g Triphenylphosphin und 5O ml Dichlormethan wurde bei -15°C und unter Stickstoffbegasung innert 1O Minuten portionenweise mit 15,6 g Tetrabrommethan versetzt. Das Reaktionsgemisch wurde 4 Stunden bei Raumtemperatur gerührt und dann eingeengt. Der Rückstand wurde in 3OO ml Hexan aufgeschlämmt, das Gemisch auf -2O°C abgekühlt und der dabei entstandene Niederschlag abgenutscht. Das Filtrat wurde eingeengt und der Rückstand an Kieselgel mit Hexan chromatographisch gereinigt. Dies ergab 11 g (E)-3-(trans-4-Pentylcyclohexyl)allylbromid.

In analoger Weise können folgende Verbindungen hergestellt werden:
trans-4-Propylcyclohexyl-(E)-3-(trans-4-methylcyclohexyl)allyl-äther;
trans-4-Propylcyclohexyl-(E)-3-(trans-4-aethylcyclohexyl)allyl-äther;
trans-4-Propylcyclohexyl-(E)-3-(trans-4-propylcyclohexyl)allyl-äther;
trans-4-Propylcyclohexyl-(E)-3-(trans-4-butylcyclohexyl)allyl-äther;
trans-4-Propylcyclohexyl-(E)-3-(trans-4-pentylcyclohexyl)allyl-äther;
trans-4-Propylcyclohexyl-(E)-3-(trans-4-hexylcyclohexyl)allyl-äther;
trans-4-Propylcyclohexyl-(E)-3-(trans-4-heptylcyclohexyl)allyl-äther;
trans-4-Pentylcyclohexyl-(E)-3-(trans-4-methylcyclohexyl)allyl-äther;
trans-4-Pentylcyclohexyl-(E)-3-(trans-4-aethylcyclohexyl)allyl-äther;
trans-4-Pentylcyclohexyl-(E)-3-(trans-4-butylcyclohexyl)allyl-äther;
trans-4-Pentylcyclohexyl-(E)-3-(trans-4-pentylcyclohexyl)allyl-äther;
trans-4-Pentylcyclohexyl-(E)-3-(trans-4-hexylcyclohexyl)allyl-äther;
trans-4-Pentylcyclohexyl-(E)-3-(trans-4-heptylcyclohexyl)allyl-äther;
trans-4-(trans-4-Propylcyclohexyl)cyclohexyl-(E)-3-(trans-4-propylcyclohexyl)allyl-äther;
trans-4-(trans-4-Propylcyclohexyl)cyclohexyl-(E)-3-(trans-4-pentylcyclohexyl)allyl-äther;
trans-4-[2-(trans-4-Propylcyclohexyl)äthyl]cyclohexyl-(E)-3-(trans-4-pentylcyclohexyl)allyl-äther, Smp. (C-S_{B}) 58°C, Klp. (S_{B}-I) 118°C.

### Beispiel 6

Ein Gemisch von 4,5 g trans-4-(trans-4-Propylcyclohexyl)cyclohexancarboxaldehyd, 5 g Aethyl-diäthylphosphonoacetat (C₂H₅O)₂PCH₂COOC₂H₅, 1,9 g Kaliumhydroxid und 1OO ml Tetrahydrofuran wurde während 15 Minuten gerührt und dann mit 5OO ml Wasser versetzt und anschliessend dreimal mit je 100 ml Diäthyläther extrahiert. Die vereinigten organischen Phasen wurden zweimal mit je 5OO ml Wasser gewaschen, über Magnesiumsulfat getrocknet, filtriert und anschliessend eingeengt. Der Rückstand wurde an Kieselgel mit Toluol chromatographisch gereinigt. Umkristallisation aus Aceton ergab 1,8 g reinen (E)-3-[trans-4-(trans-4-Propylcyclohexyl)cyclohexyl]acrylsäureäthylester mit Smp. (C-N) 33°C und Klp. (N-I) 76°C.

Der als Ausgangsmaterial verwendete trans-4-(trans-4-Propylcyclohexyl)cyclohexancarboxaldehyd wurde wie folgt hergestellt:

Eine Lösung von 5 g trans-4-(trans-4-Propylcyclohexyl)cyclohexancarbonitril in 5O ml Dichlormethan wurde unter Stickstoffbegasung bei -78°C tropfenweise mit 6O ml einer 20%-igen Lösung (Gew./Vol.) von Diisobutylaluminiumhydrid in Hexan versetzt. Das Reaktionsgemisch wurde noch 2 Stunden bei Raumtemperatur gerührt, dann mit 5OO ml Wasser versetzt und anschliessend viermal mit je 2OO ml Dichlormethan extrahiert. Die vereinigten organischen Phasen wurden zweimal mit 5OO ml Wasser gewaschen, über Magnesiumsulfat getrocknet, filtriert und anschliessend eingeengt. Dies ergab 4,5 g trans-4-(trans-4-Propylcyclohexyl)cyclohexancarboxaldehyd.

In analoger Weise können folgende Verbindungen hergestellt werden:
(E)-3-[trans-4-(trans-4-Propylcyclohexyl)cyclohexyl]acrylsäure-methylester;
(E)-3-[trans-4-(trans-4-Propylcyclohexyl)cyclohexyl]acrylsäure-propylester;
(E)-3-[trans-4-(trans-4-Propylcyclohexyl)cyclohexyl]acrylsäure-butylester;
(E)-3-[trans-4-(trans-4-Propylcyclohexyl)cyclohexyl]acrylsäure-pentylester;
(E)-3-[trans-4-(trans-4-Butylcyclohexyl)cyclohexyl]acrylsäure-methylester;
(E)-3-[trans-4-(trans-4-Butylcyclohexyl)cyclohexyl]acrylsäure-aethylester;
(E)-3-[trans-4-(trans-4-Butylcyclohexyl)cyclohexyl]acrylsäure-propylester;
(E)-3-[trans-4-(trans-4-Butylcyclohexyl)cyclohexyl]acrylsäure-butylester;
(E)-3-[trans-4-(trans-4-Butylcyclohexyl)cyclohexyl]acrylsäure-pentylester;
(E)-3-[trans-4-(trans-4-Pentylcyclohexyl)cyclohexyl]acrylsäure-methylester;
(E)-3-[trans-4-(trans-4-Pentylcyclohexyl)cyclohexyl]acrylsäure-aethylester, Smp. (C-S_{B}) 27°C, S_{B}-N 49°C, Klp. (N-I) 93°C;
(E)-3-[trans-4-(trans-4-Pentylcyclohexyl)cyclohexyl]acrylsäure-propylester;
(E)-3-[trans-4-(trans-4-Pentylcyclohexyl)cyclohexyl]acrylsäure-butylester;
(E)-3-[trans-4-(trans-4-Pentylcyclohexyl)cyclohexyl]acrylsäure-pentylester.

## Patentansprüche

1. Verbindungen der allgemeinen Formel 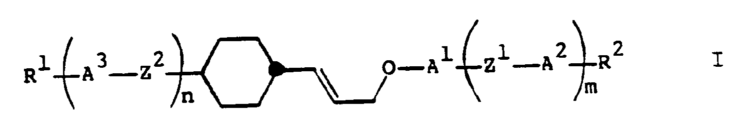 worin R¹ eine Gruppe R³ oder R³-A⁴-Z³- und R² eine Gruppe R⁴ oder -Z⁴-A⁵-R⁴ bezeichnen; m und n unabhängig voneinander für die Zahl O oder 1 stehen; A¹, A², A³, A⁴ und A⁵ unabhängig voneinander unsubstituiertes oder mit Halogen, Cyano und/oder Methyl substituiertes 1,4-Phenylen, in welchem gegebenenfalls 1 oder 2 CH-Gruppen durch Stickstoff ersetzt sind, trans-1,4-Cyclohexylen, in welchem gegebenenfalls 2 nicht benachbarte Methylengruppen durch Sauerstoff und/oder Schwefel ersetzt sind, 1-Cyano-trans-1,4-cyclohexylen, Bicyclo[2.2.2]octan-1,4-diyl, Naphthalin-2,6-diyl, Tetralin-2,6-diyl oder trans-Decalin-2,6-diyl darstellen; Z¹, Z², Z³ und Z⁴ unabhängig voneinander eine einfache Kovalenzbindung, -COO-, -OOC-, -CH₂O-, -OCH₂-, -CH₂CH₂-, -C=C-, -(CH₂)₃O-, -O(CH₂)₃- oder die trans-Form von -CH=CH-CH_{2O}- oder -OCH₂-CH=CH- bedeuten; R₃ und R₄ unabhängig voneinander Halogen, Cyano, -NCS, -CF₃, -OCF₃ oder Alkyl bezeichnen, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist; und X Halogen, Cyano oder Methyl bedeutet.

2. Verbindungen nach Anspruch 1, dadurch gekennzeichnet, dass A¹ unsubstituiertes oder mit Halogen, Cyano und/oder Methyl substituiertes 1,4-Phenylen oder trans-1,4-Cyclohexylen darstellt.

3. Verbindungen nach Anspruch 1, gekennzeichnet durch die allgemeine Formel 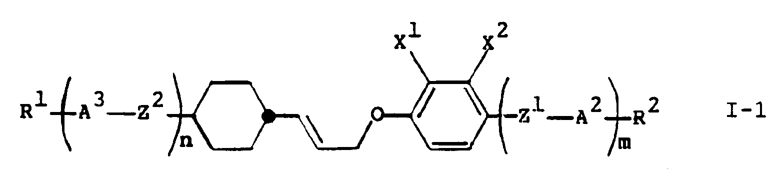 worin X¹ und X² unabhängig voneinander Wasserstoff, Halogen, Cyano oder Methyl bezeichnen und A², A³, R¹, R², Z¹, Z², m und n die in Anspruch 1 gegebenen Bedeutungen haben.

4. Verbindungen nach einem der Ansprüche 1 bis 3, dadurch gekennzeichnet, dass A³ trans-1,4-Cyclohexylen darstellt, Z² eine einfache Kovalenzbindung oder -CH₂CH₂- bedeutet, und n für die Zahl O oder 1 steht.

5. Verbindungen nach einem der Ansprüche 1 bis 4, dadurch gekennzeichnet, dass Z¹, Z², Z³ und Z⁴ je eine einfache Kovalenzbindung bedeuten oder eine der Gruppen Z¹, Z², Z³ und Z⁴ auch -COO-, -OOC-, -CH₂O-, -OCH₂-, -CH₂CH₂-, -C=C-, -(CH₂)₃O-, -O(CH₂)₃- oder die trans-Form von -CH=CH-CH₂O- oder -OCH₂-CH=CH- bedeutet.

6. Verbindungen nach Anspruch 1, gekennzeichnet durch die allgemeine Formel 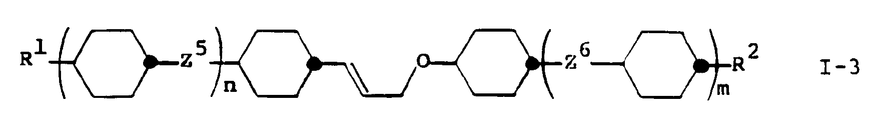 worin Z⁵ und Z⁶ unabhängig voneinander eine einfache Kovalenzbindung oder -CH₂CH₂- bezeichnen, und R¹, R², m und n die in Anspruch 1 gegebenen Bedeutungen haben.

7. Verbindungen nach einem der Ansprüche 1 bis 6, dadurch gekennzeichnet, dass R¹ eine Gruppe R³ bedeutet, R² eine Gruppe R⁴ bedeutet, und entweder m und n für die Zahl O stehen, oder m für die Zahl 1 und n für die Zahl O stehen oder m für die Zahl O und n für die Zahl 1 stehen.

8. Verbindungen nach Anspruch 1, gekennzeichnet durch die allgemeinen Formeln 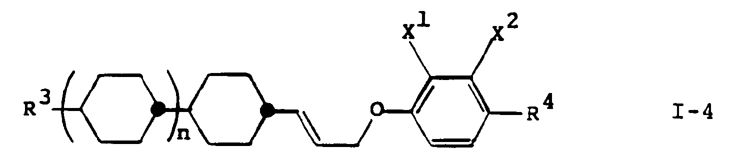 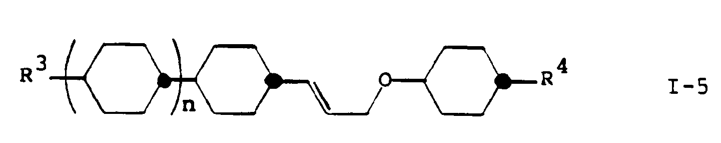 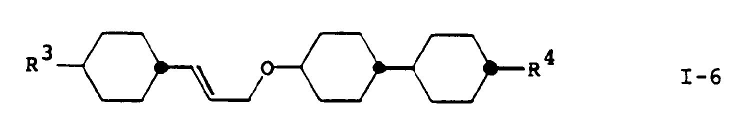 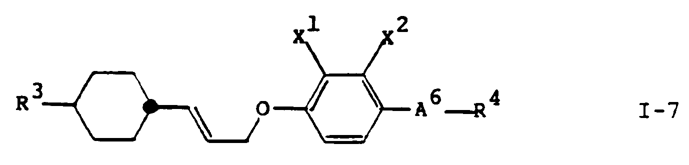 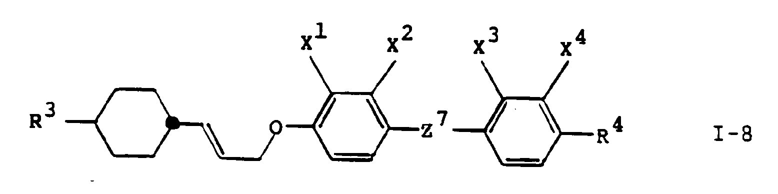 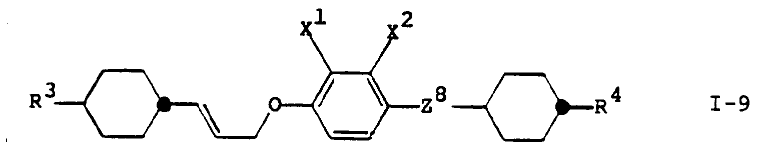 worin n für die Zahl O oder 1 steht; X¹, X², X³ und X⁴ unabhängig voneinander Wasserstoff, Halogen, Cyano oder Methyl bedeuten; A⁶ 1,4-Phenylen, Pyridin-2,5-diyl, Pyrimidin-2,5-diyl, Pyrazin-2,5-diyl, trans-1,4-Cyclohexylen, trans-1,3-Dioxan-2,5-diyl oder trans-Decalin-2,6-diyl darstellt; Z⁷ eine einfache Kovalenzbindung, -COO- oder -OOC- bedeutet; Z⁸ eine einfache Kovalenzbindung, -OOC-, -OCH₂-, -C≡C-, -O(CH₂)₃- oder die trans-Form von -OCH₂-CH=CH- bezeichnet; und R³ und R⁴ die in Anspruch 1 gegebenen Bedeutungen haben.

9. Verbindungen nach einem der Ansprüche 1 bis 8, dadurch gekennzeichnet, dass R³ und R⁴ höchstens je 18 Kohlenstoffatome aufweisen und höchstens einer der Reste R³ und R⁴ Halogen, Cyano, -NCS, -CF₃ oder -OCF₃ bedeutet.

10. Verbindungen nach einem der Ansprüche 1 bis 9, dadurch gekennzeichnet, dass R³ Alkyl, Alkenyl, Alkoxy, Alkenyloxy, Alkoxycarbonyl, Alkenyloxycarbonyl, Alkanoyloxy oder Alkenoyloxy, vorzugsweise Alkyl oder Alkenyl bedeutet.

11. Verbindungen nach einem der Ansprüche 1 bis 10, dadurch gekennzeichnet, dass R⁴ Alkyl, Alkenyl, Alkoxy, Alkenyloxy, Alkoxycarbonyl, Alkenyloxycarbonyl, Alkanoyl oxy, Alkenoyloxy, Halogen, Cyano, -NCS, -CF₃ oder -OCF₃, vorzugsweise Alkyl, Alkenyl, Alkoxy, Alkenyloxy, Fluor, Chlor, Cyano, -NCS, -CF₃ oder -OCF₃ bedeutet.

12. Flüssigkristallines Gemisch mit mindestens 2 Komponenten, dadurch gekennzeichnet, dass mindestens eine Komponente eine Verbindung der in Anspruch 1 definierten Formel I ist.

13. Verwendung der Verbindungen der in Anspruch 1 definierten Formel I für elektro-optische Zwecke.

14. Verbindungen der allgemeinen Formel 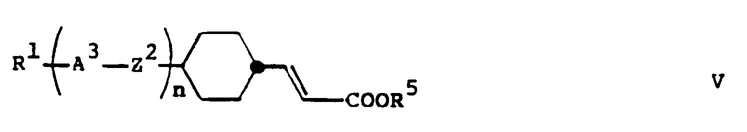 worin R¹ eine Gruppe R³ oder R³-A⁴-Z³- bezeichnet; n für die Zahl 1 oder, wenn R¹ eine Gruppe R³-A⁴-Z²- bezeichnet, auch für die Zahl O steht; A³ und A⁴ unabhängig voneinander unsubstituiertes oder mit Halogen, Cyano, und/oder Methyl substituiertes 1,4-Phenylen, in welchem gegebenenfalls 1 oder 2 CH-Gruppen durch Stickstoff ersetzt sind, trans-1,4-Cyclohexylen, in welchem gegebenenfalls 2 nicht benachbarte Methylengruppen durch Sauerstoff und/oder Schwefel ersetzt sind, 1-Cyano-trans-1,4-cyclohexylen, Bicyclo[2.2.2]octan-1,4-diyl, Naphthalin-2,6-diyl, Tetralin-2,6-diyl oder trans-Decalin-2,6-diyl darstellen; Z² und Z³ unabhängig voneinander eine einfache Kovalenzbindung, -COO-, -OOC-, -CH₂O-, -OCH₂-, -CH₂CH₂-, -C≡C-, -(CH₂)₃O-, -O(CH₂)₃- oder die trans-Form von -CH=CH-CH₂O- oder -OCH₂-CH=CH- bedeuten; R³ Halogen, Cyano, -NCS, -CF₃, -OCF₃ oder Alkyl bezeichnet, in welchem gegebenenfalls eine >CH-CH< durch >C=C< ersetzt ist und/oder gegebenenfalls eine oder zwei nicht benachbarte Methylengruppen durch -O-, -COO- und/ oder -OOC- ersetzt sind und/oder gegebenenfalls eine Methylengruppe durch -CHX- ersetzt ist; X Halogen, Cyano oder Methyl bedeutet; und R⁵ Alkyl bezeichnet.

15. Verbindungen nach Anspruch 14 gekennzeichnet durch die allgemeine Formel 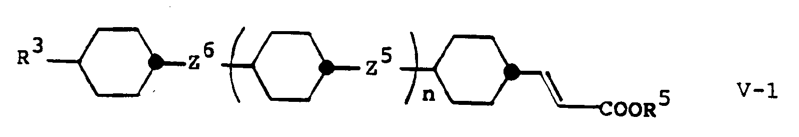 worin n für die Zahl O oder 1 steht, Z⁵ und Z⁶ unabhängig voneinander eine einfache Kovalenzbindung oder -CH₂CH₂- darstellen, und R³ und R⁵ die in Anspruch 14 gegebenen Bedeutungen haben.

## Claims

1. Compounds of the general formula 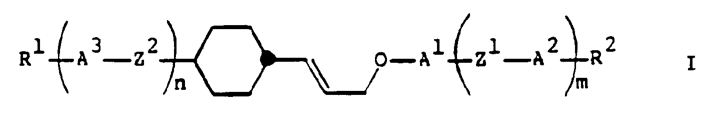 wherein R¹ denotes a group R³ or R³-A⁴-Z³and R² denotes a group R⁴ or -Z⁴-A⁵-R⁴; m and n each individually stand for the number O or 1; A¹, A², A³, A⁴ and A⁵ each independently represent unsubstituted or halogen-, cyano- and/or methyl-substituted 1,4-phenylene in which optionally 1 CH group or 2 CH groups is/are replaced by nitrogen, trans-1,4-cyclohexylene in which optionally 2 non-adjacent methylene groups are replaced by oxygen and/or sulphur, 1-cyano-trans-1,4-cyclohexylene, bicyclo[2.2.2]octane-1,4-diyl, naphthalene-2,6-diyl, tetralin-2,6-diyl or trans-decalin-2,6-diyl; Z¹, Z², Z³ and Z⁴ each independently signify a single covalent bond, -COO-, -OOC-, -CH₂O-, -OCH₂-, -CH₂CH₂-, -C≡C-, -(CH₂)₃O-, -O(CH₂)₃- or the trans form of -CH=CH-CH₂O- or -OCH₂-CH=CH-; R³ and R⁴ each independently denote halogen, cyano, -NCS, -CF₃, -OCF₃ or alkyl in which optionally one >CH-CH< is replaced by >C=C< and/or optionally one methylene group or two non-adjacent methylene groups is/are replaced by -O-, -COO- and/or -OOC- and/or optionally one methylene group is replaced by -CHX-; and X signifies halogen, cyano or methyl.

2. Compounds according to claim 1, characterized in that A¹ represents unsubstituted or halogen-, cyano and/or methyl-substituted 1,4-phenylene or trans-1,4-cyclohexylene.

3. Compounds according to claim 1, characterized by the general formula 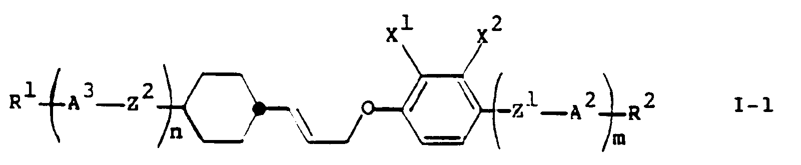 wherein X¹ and X² each independently denote hydrogen, halogen, cyano or methyl and A², A³, R¹, R², Z¹, Z², m and n have the significances given in claim 1.

4. Compounds according to any one of claims 1 to 3, characterized in that A³ represents trans-1,4-cyclohexylene, Z² signifies a single covalent bond or -CH₂CH₂- and n stands for the number O or 1.

5. Compounds according to any one of claims 1 to 4, characterized in that Z¹, Z², Z³ and Z⁴ each signify a single covalent bond or one of the groups Z¹, Z², Z³ and Z⁴ also signifies -COO-, -OOC-, -CH₂O-, -OCH₂-, -CH₂CH₂-, -C≡C-, -(CH₂)₃O-, -O(CH₂)₃- or the trans form of -CH=CH-CH₂O- or -OCH₂-CH=CH-.

6. Compounds according to claim 1, characterized by the general formula 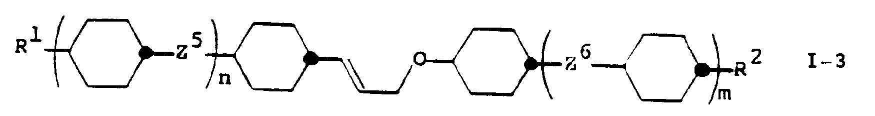 wherein Z⁵ and Z⁶ each independently denote a single covalent bond or -CH₂CH₂- and R¹, R², m and n have the significances given in claim 1.

7. Compounds according to any one of claims 1 to 6, characterized in that R¹ signifies a group R³, R² signifies a group R⁴ and either m and n stand for the number O or m stands for the number 1 and n stands for the number O or m stands for the number O and n stands for the number 1.

8. Compounds according to claim 1, characterized by the general formulae 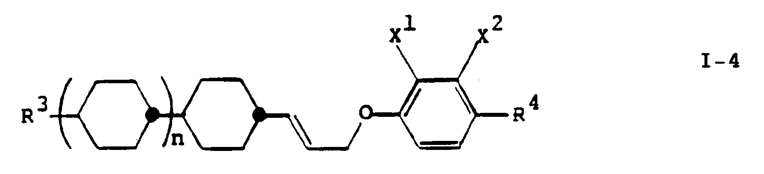 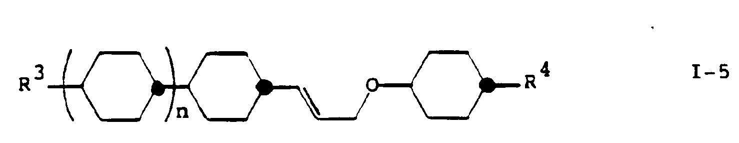 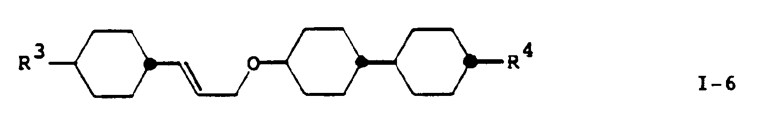 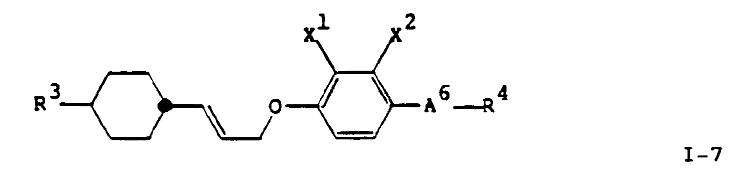 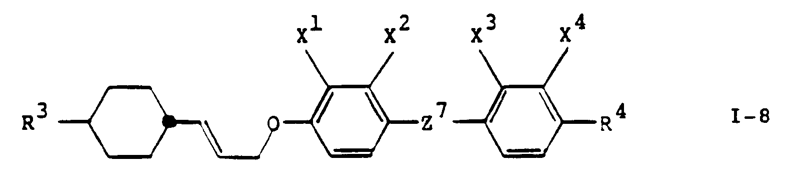 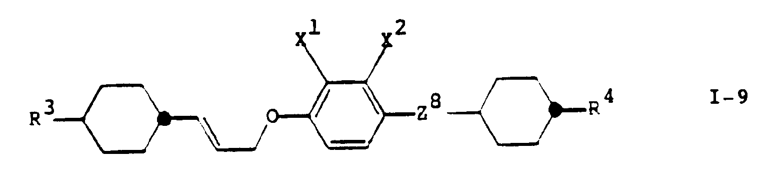 wherein n stands for the number O or 1; X¹, X², X³ and X⁴ each independently signify hydrogen, halogen, cyano or methyl; A⁶ represents 1,4-phenylene, pyridine-2,5-diyl, pyrimidine-2,5-diyl, pyrazine-2,5-diyl, trans-1,4-cyclohexylene, trans-1,3-dioxane-2,5-diyl or trans-decalin-2,6-diyl; Z⁷ signifies a single covalent bond, -COO- or -OOC-; Z⁸ denotes a single covalent bond, -OOC-, -OCH₂-, -C≡C-, -O(CH₂)₃- or the trans form of -OCH₂-CH=CH-; and R³ and R⁴ have the significances given in claim 1.

9. Compounds according to any one of claims 1 to 8, characterized in that R³ and R⁴ have a maximum of in each case 18 carbon atoms and a maximum of one of the residues R³ and R⁴ signifies halogen, cyano, -NCS, -CF₃ or -OCF₃.

10. Compounds according to any one of claims 1 to 9, characterized in that R³ signifies alkyl, alkenyl, alkoxy, alkenyloxy, alkoxycarbonyl, alkenyloxycarbonyl, alkanoyloxy or alkenoyloxy, preferably alkyl or alkenyl.

11. Compounds according to any one of claims 1 to 10, characterized in that R⁴ signifies alkyl, alkenyl, alkoxy, alkenyloxy, alkoxycarbonyl, alkenyloxycarbonyl, alkanoyloxy, alkenoyloxy, halogen, cyano, -NCS, -CF₃ or -OCF₃, preferably alkyl, alkenyl, alkoxy, alkenyloxy, fluorine, chlorine, cyano, -NCS, -CF₃ or -OCF₃.

12. A liquid crystalline mixture having at least 2 components, characterized in that at least one component is a compound of formula I defined in claim 1.

13. The use of the compounds of formula I defined in claim 1 for electro-optical purposes.

14. Compounds of the general formula 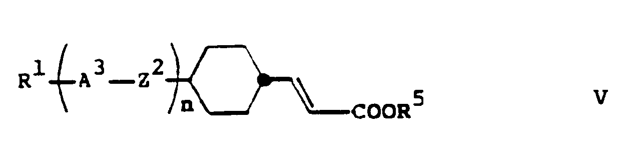 wherein R¹ denotes a group R³ or R³-A⁴-Z³-; n stands for the number 1 or, when R¹ denotes a group R³-A⁴-Z²-, also for the number O; A³ and A⁴ each independently represent unsubstituted or halogen-, cyano- and/or methyl-substituted 1,4-phenylene in which optionally 1 CH group or 2 CH groups is/are replaced by nitrogen, trans-1,4-cyclohexylene in which optionally 2 non-adjacent methylene groups are replaced by oxygen and/or sulphur, 1-cyano-trans-1,4-cyclohexylene, bicyclo[2.2.2]octane-1,4-diyl, naphthalene-2,6-diyl, tetralin-2,6-diyl or trans-decalin-2,6-diyl; Z² and Z³ each independently signify a single covalent bond, -COO-, -OOC-, -CH₂O-, -OCH₂-, -CH₂CH₂-, -C≡C-, -(CH₂)₃O-, -O(CH₂)₃- or the trans form of -H=CH-CH₂O- or -OCH₂-CH=CH-; R³ denotes halogen, cyano, -NCS, -CF₃, -OCF₃ or alkyl in which optionally one >CH-CH< is replaced by >C=C< and/or optionally one methylene group or two non-adjacent methylene groups is/are replaced by -O-, -COO- and/or -OOC- and/or optionally one methylene group is replaced by -CHX-; X signifies halogen, cyano or methyl; and R⁵ denotes alkyl.

15. Compounds according to claim 14, characterized by the general formula 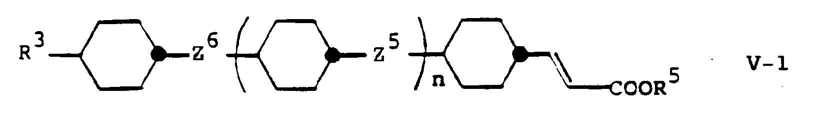 wherein n stands for the number O or 1, Z⁵ and Z⁶ each independently represent a single covalent bond or -CH₂CH₂- and R³ and R⁵ have the significances given in claim 14.

## Revendications

1. Composés de formule générale 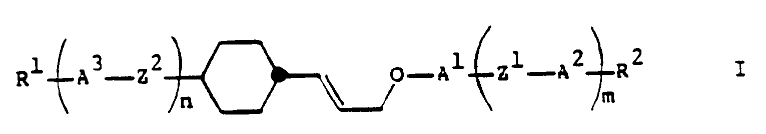 dans laquelle R¹ représente un groupe R³ ou R³-A⁴-Z³- et R² représente un groupe R⁴ ou -Z⁴-A⁵-R⁴; m et n sont égaux chacun, indépendamment l'un de l'autre, à 0 ou 1; A¹, A², A³, A⁴ et A⁵ représentent chacun, indépendamment les uns des autres, un groupe 1,4-phénylène non substitué ou substitué par un halogène, un groupe cyano et/ou méthyle et dans lequel le cas échéant 1 ou 2 groupes CH sont remplacés par l'azote, un groupe trans-1,4-cyclohexylène dans lequel le cas échéant 2 groupes méthylène non voisins sont remplacés par l'oxygène et/ou le soufre, un groupe 1-cyano-trans-1,4-cyclohexylène, bicyclo[2.2.2]octane-1,4-diyle, naphtalène-2,6-diyle, tétraline-2,6-diyle, ou trans-décaline-2,6-diyle; Z¹, Z², Z³ et Z⁴ représentent chacun, indépendamment les uns des autres, une liaison covalente simple, un groupe -COO-, -OOC-, -CH₂O-, -OCH₂-, -CH₂CH₂-; C=C-, -(CH₂)₃O-, -O(CH₂)₃- ou la forme trans du groupe -CH=CH-CH₂O- ou -OCH₂-CH=CH-; R³ et R⁴ représentent chacun, indépendamment l'un de l'autre, un halogène, un groupe cyano, -NCS, -CF₃, -OCF₃ ou un groupe alkyle dans lequel le cas échéant un groupe <CH-CH< est remplacé >C=C< et/ou le cas échéant un ou deux groupes méthylène non voisins sont remplacés par -O-, -COO- et/ou -OOC- et/ou le cas échéant un groupe méthylène est remplacé par -CHX-; et X représente un halogène, un groupe cyano ou méthyle.

2. Composés selon revendication 1, caractérisés en ce que A¹ représente un groupe 1,4-phénylène ou trans-1,4-cyclohexylène non substitué ou substitué par un halogène, un groupe cyano et/ou un groupe méthyle.

3. Composés selon revendication 1, caractérisés par la formule générale 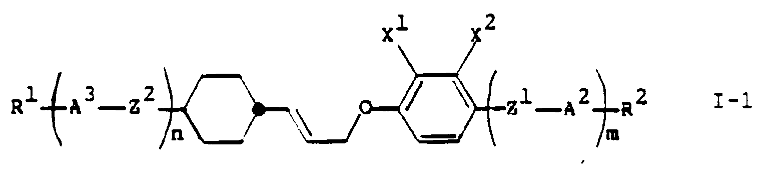 dans laquelle X¹ et X² représentent chacun, indépendamment l'un de l'autre, l'hydrogène, un halogène, un groupe cyano ou méthyle et A², A³, R¹, R², Z¹, Z², m et n ont les significations indiquées dans la revendication 1.

4. Composés selon l'une des revendications 1 à 3, caractérisés en ce que A³ représente un groupe trans-1,4-cyctohexylène, Z² une liaison covalente simple ou un groupe -CH₂CH₂-, et n est égal à 0 ou 1.

5. Composés selon l'une des revendications 1 à 4, caractérisés en ce que Z¹, Z², Z³ et Z⁴ représentent chacun une liaison covalente simple ou bien encore l'un des symboles Z¹, Z², Z³ et Z⁴ représente un groupe -COO-, -OOC-, -CH₂O-, -OCH₂-, -CH₂CH₂-, -C=C-, -(CH₂)₃O-, -O(CH₂)₃- ou la forme trans du groupe -CH=CH-CH₂O- ou -OCH₂-CH=CH-.

6. Composés selon revendication 1, caractérisés par la formule générale 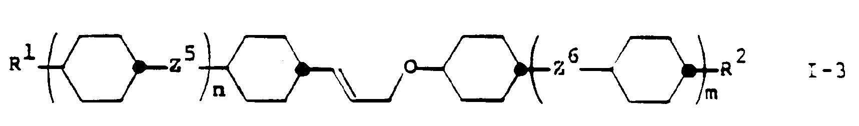 dans laquelle Z⁵ et Z⁶ représentent chacun, indépendamment l'un de l'autre, une liaison covalente simple ou un groupe -CH₂CH₂-, et R¹, R², m et n ont les significations indiquées dans la revendication 1.

7. Composés selon une des revendications 1 à 6, caractérisés en ce que R¹ représente un groupe R³, R² représente un groupe R⁴, et m et n sont égaux à 0 ou bien m est égal à 1 et n à 0 ou bien m est égal à 0 et n à 1.

8. Composés selon revendication 1, caractérisés par les formules générales 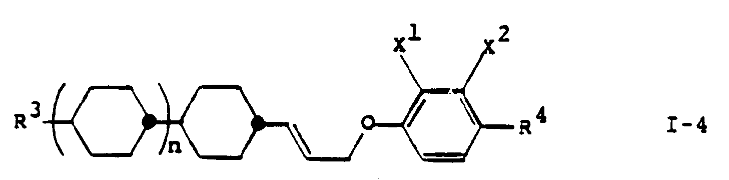 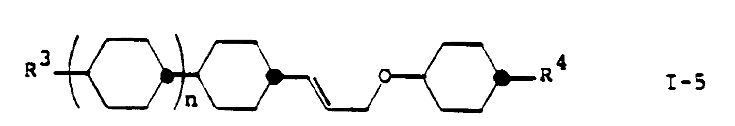 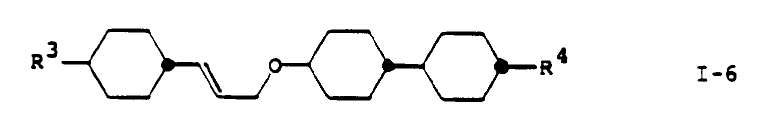 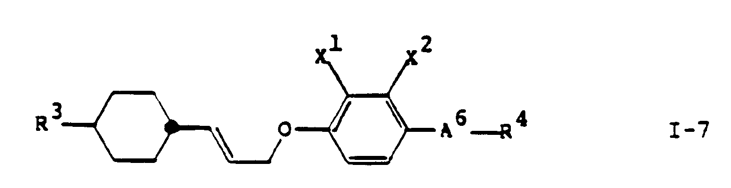 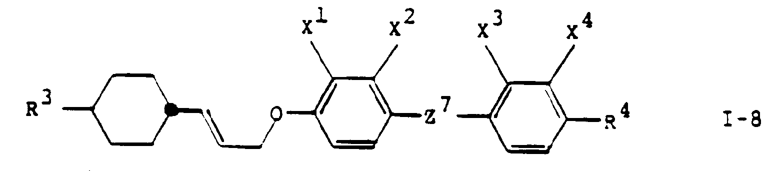 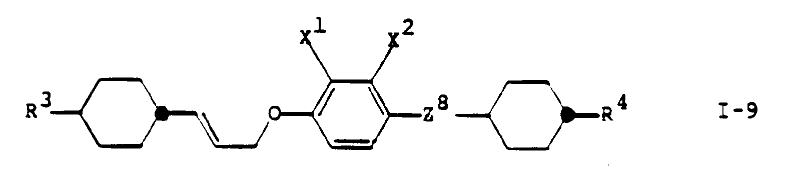 dans lesquelles n est égal à 0 ou 1; X¹, X², X³ et X⁴ représentent chacun, indépendamment les uns des autres, l'hydrogène, un halogène, un groupe cyano ou méthyle; A⁶ représente un groupe 1,4-phénylène, pyridine-2,5diyle, pyrimidine-2,5-diyle, pyrazine-2,5-diyle, trans-1,4-cyclohexylène, trans-1,3-dioxanne-2,5-diyle ou trans-décaline-2,6-diyle; Z⁷ représente une liaison covalente simple, un groupe -COO- ou -OOC-; Z⁸ représente une liaison covalente simple, un groupe -OOC-, -OCH₂-, -C≡C-, -O(CH₂)₃- ou la forme trans du groupe -OCH₂-CH=CH-; et R³ et R⁴ ont les significations indiquées dans la revendication 1.

9. Composés selon une des revendications 1 à 8, caractérisés en ce que R³ et R⁴ contiennent chacun au maximum 18 atomes de carbone et un seulement au maximum des symboles R³ et R⁴ représente un halogène, un groupe cyano, -NCS, -CF₃ ou -OCF₃.

10. Composés selon une des revendications 1 à 9, caractérisés en ce que R³ représente un groupe alkyle, alcényle, alcoxy, alcényloxy, alcoxycarbonyle, alcényloxycarbonyle, alcanoyloxy ou alcénoyloxy, de préférence un groupe alkyle ou alcényle.

11. Composés selon une des revendications 1 à 10, caractérisés en ce que R⁴ représente un groupe alkyle, alcényle, alcoxy, alcényloxy, alcoxycarbonyle, alcényloxycarbonyle, alcanoyloxy ou alcénoyloxy, un halogène, un groupe cyano, -NCS, -CF₃ ou -OCF₃, de préférence un groupe alkyle, alcényle, alcoxy, alcényloxy, le fluor, le chlore, un groupe cyano, -NCS, -CF₃ ou -OCF₃.

12. Mélange à cristaux liquides à au moins deux composants, caractérisé en ce qu'un composant au moins consiste en un composé de formule I définie dans la revendication 1.

13. Utilisation des composés de formule I définie dans la revendication 1, dans des applications électro-optiques.

14. Composés de formule générale 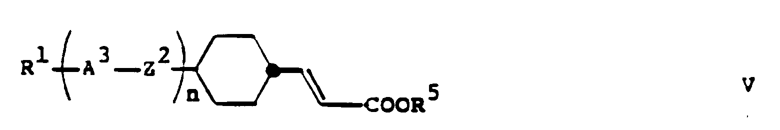 dans laquelle R¹ représente un groupe R³ ou R³-A⁴-Z³-; n est égal à 1, ou bien encore, lorsque R¹ représente un groupe R³-A⁴-Z²-, à 0; A³ et A⁴ représentent chacun, indépendamment l'un de l'autre, un groupe 1,4-phénylène non substitué ou substitué par un halogène, un groupe cyano et/ou méthyle et dans lequel le cas échéant 1 ou 2 groupes CH sont remplacés par l'azote, un groupe trans-1,4-cyclohexylène dans lequel le cas échéant 2 groupes méthylène non voisins sont remplacés par l'oxygène et/ou le soufre, un groupe 1-cyano-trans-1,4-cyclohexylène, bicyclo[2.2.2]octane-1,4-diyle, naphtalène-2,6-diyle, tétraline-2,6-diyle ou trans-décaline-2,6-diyle; Z² et Z³ représentent chacun, indépendamment l'un de l'autre, une liaison covalente simple, un groupe -COO-, -OOC-, -CH₂O-, -OCH₂-, -CH₂CH₂-, -C≡C-, -(CH₂)₃O-, -O(CH₂)₃- ou la forme trans du groupe -CH=CH-CH₂O- ou -OCH₂-CH=CH-; R³ représente un halogène, un groupe cyano, -NCS, -CF₃, -OCF₃ ou un groupe alkyle dans lequel le cas échéant un motif >CH-CH< est remplacé par >C=C< et/ou le cas échéant 1 ou 2 groupes méthylène non voisins sont remplacés par -O-, -COO- et/ou -OOC- et/ou le cas échéant un groupe méthylène est remplacé par -CHX-; X représente un halogène, un groupe cyano ou méthyle; et R⁵ un groupe alkyle.

15. Composés selon revendication 14, caractérisés par la formule générale 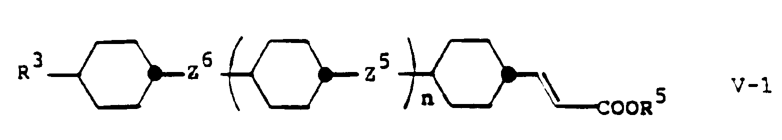 dans laquelle n est égal à 0 ou 1, Z⁵ et Z⁶ représentent chacun, indépendamment l'un de l'autre, une liaison covalente simple ou un groupe -CH₂CH₂-, et R³ et R⁵ ont les significations indiquées dans la revendication 14.
